(19) Europäisches Patentamt
European Patent Office
Office européen des brevets

(11) **EP 3 858 851 B1**

(12) **EUROPEAN PATENT SPECIFICATION**

(45) Date of publication and mention
of the grant of the patent:
**01.04.2026 Bulletin 2026/14**

(21) Application number: **19867810.4**

(22) Date of filing: **30.09.2019**

(51) International Patent Classification (IPC):
***D01F 4/02*** *(2006.01)*     ***D01F 11/02*** *(2006.01)*
***D01D 5/06*** *(2006.01)*

(52) Cooperative Patent Classification (CPC):
**D01F 4/00; D01D 5/06; D01F 4/02; D01F 11/02**

(86) International application number:
**PCT/JP2019/038614**

(87) International publication number:
**WO 2020/067572 (02.04.2020 Gazette 2020/14)**

(54) **PROTEIN COMPOSITION PRODUCTION METHOD**

HERSTELLUNGSVERFAHREN EINER PROTEINZUSAMMENSETZUNG

PROCÉDÉ DE PRODUCTION DE COMPOSITION DE PROTÉINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.09.2018 JP 2018185614
27.09.2019 JP 2019177553**

(43) Date of publication of application:
**04.08.2021 Bulletin 2021/31**

(73) Proprietor: **Spiber Inc.
Tsuruoka-shi, Yamagata 997-0052 (JP)**

(72) Inventors:
• **ASAKURA, Tetsuo
Fuchu-shi, Tokyo 183-8538 (JP)**
• **ABE, Yunosuke
Tsuruoka-shi, Yamagata 997-0052 (JP)**
• **ISHII, Hideto
Tsuruoka-shi, Yamagata 997-0052 (JP)**

(74) Representative: **Handley, Matthew Edward et al
Venner Shipley LLP
St James's
79 Oxford Street
Manchester M1 6EG (GB)**

(56) References cited:
**WO-A1-02/052099**     **WO-A1-2012/165476**
**WO-A1-2018/164020**     **JP-A- 2007 177 370**
**JP-A- 2010 236 149**     **JP-A- H04 263 614**
**JP-A- H10 298 201**

Remarks:
The complete document including Reference
Table(s) and the Sequence Listing(s) can be
downloaded from the EPO website

## Description

Technical Field

The present invention relates to a production method of a protein composition.

Background Art

A spinning method using an acid such as formic acid is widely used. For example, Patent Literature 1 discloses a method of treating a biological sample containing a structural polypeptide with an acid. WO 02/052099 describes a process for producing a regenerated fiber by treating a regenerated collagen fiber with a monofunctional epoxy compound and a metallic aluminum salt. JP 2007 177370 describes a hair fiber bundle comprising regenerative collagen fiber and human hair. WO 2012/165476 describes an artificial fiber that has a stress of at least 350 MPa and a toughness of at least 138 MJ/m$^3$.

EP 0 488 687 A2 discloses a process for spinning polypeptide fibers from spinning solutions containing a polypeptide and a mixture of formic acid and at least one lithium halide.

Citation List

Patent Literature

Patent Literature 1: JP 2004-503204 A

Summary of Invention

Technical Problem

The present inventors found that in a protein fiber produced using a dope solution (spinning raw material solution) in which a carboxylic acid such as formic acid is used as a solvent, an ester group is formed by a dehydration condensation reaction between a hydroxyl group in a protein and the carboxylic acid during spinning. The present inventors further found that in the protein fiber obtained as described above, hydrolysis of the ester group added to the protein proceeds with a trace amount of the carboxylic acid, such as formic acid, remaining on a surface or inside of the protein as a catalyst, the carboxylic acid is thus isolated, and the isolated carboxylic acid causes an odor or the like. The present invention is provided to solve such problems newly found by the present inventors.

That is, an object of the present invention is to provide a protein composition in which an ester group included in a protein is removed or reduced, a production method of the same, and an ester group removal method.

Solution to Problem

The present invention is defined in the claims. The technical disclosure set out below may in some respects go beyond the scope of the claimed invention, and elements of the disclosure which do not fall within the scope of the claims are provided for information, to place the actual invention claimed in a broader technical context

Advantageous Effects of Invention

According to the present invention, a production method of a protein composition in which an ester group included in a protein is removed or reduced, and an ester group removal method are provided. By performing a hydrolysis treatment on the ester group in the protein composition, a shrink-proof treatment effect of the protein composition is also exhibited at the same time. That is, in the protein composition according to the present invention, the ester group is removed or reduced, and the shrinkage when being brought into contact with water is also reduced.

Brief Description of Drawings

Fig. 1 is a schematic diagram illustrating an example of a domain sequence of modified fibroin.
Fig. 2 is a schematic diagram illustrating an example of a domain sequence of modified fibroin.
Fig. 3 is a schematic diagram illustrating an example of a domain sequence of modified fibroin.

Fig. 4 is an explanation diagram schematically illustrating an example of a spinning apparatus for producing a raw material fiber.

Fig. 5 is an explanation diagram schematically illustrating an example of a production apparatus for producing a protein fiber.

Fig. 6 is an explanation diagram schematically illustrating an example of a production apparatus for producing a protein fiber.

Fig. 7 is an explanation diagram schematically illustrating an example of a production apparatus for producing a protein fiber.

Fig. 8 is an explanation diagram schematically illustrating speed control means and temperature control means which can be provided in a high temperature heating furnace of Fig. 7.

Fig. 9 illustrates photographs obtained by observing a crimp state immediately after a hydrolysis treatment of the protein fiber.

Fig. 10 is a graph obtained by plotting an absorbance ratio (P1/P2) in which a residual amount of the ester group is reflected against the number of contact days between the protein fiber and water vapor, in which P1: a peak height of 1,725 cm$^{-1}$ (peak based on C=O of ester), and P2: a peak height of 1,445 cm$^{-1}$ (peak based on amide III of protein).

Fig. 11 is a spectrum diagram of FT-IR of the protein fiber immediately after the hydrolysis treatment of the ester group (1,730 cm$^{-1}$: peak based on C=O of ester).

Description

Hereinafter, embodiments of the present invention will be described in detail. However, the present invention is not limited to the following embodiments.

[Production method of protein composition]

A production method of a protein composition according to the present invention includes a step of hydrolyzing an ester group by bringing a raw material composition containing an esterified protein (a protein having an ester group) into contact with an acidic or basic (alkaline) medium.

In the present disclosure, the raw material composition may be at least one selected from the group consisting of a fiber (raw material fiber), a film (a raw material film), a molded article (a raw material molded article), a gel (raw material gel), a porous body (a raw material porous body), and a particle (raw material particle).

In the present disclosure, the raw material composition contains an esterified protein. In the present specification, the "esterified protein" refers to a protein having an ester group formed by ester-bonding a hydroxyl group of the protein and a carboxylic acid. The esterified protein may contain formic acid ester, acetic acid ester, propionic acid ester, and the like, and the esterified protein preferably contains formic acid ester.

(Protein)

Examples of the protein according to the present disclosure (hereinafter, also referred to as a "protein to be targeted") can include a natural protein and a recombinant protein (artificial protein). In addition, an example of the recombinant protein can include any protein that can be produced in an industrial scale, and examples thereof can include a protein that can be used for industrial purposes, a protein that can be used for medical purposes. According to the present invention, the protein is a structural protein. The structural protein refers to a protein forming or maintaining a structure, morphology, or the like in a living body. The structural protein may be fibroin. Specific examples of the structural protein can include spider silk fibroin, silk fibroin, keratin, collagen, elastin, resilin, and proteins derived from them. The structural protein may be, for example, modified fibroin described below, and modified spider silk fibroin is preferred, from the viewpoint of obtaining heat retaining properties, hygroscopic and exothermic properties, and/or flame retardancy.

The modified fibroin is a protein containing a domain sequence represented by Formula 1: [(A)$_n$ motif-REP]$_m$ or Formula 2: [(A)$_n$ motif-REP]$_m$- (A)$_n$ motif. An amino acid sequence (N-terminal sequence and C-terminal sequence) may be further added to either or both of the N-terminus and the C-terminus of the domain sequence of the modified fibroin. The N-terminal sequence and the C-terminal sequence, although not limited thereto, are typically regions that do not have repetitions of amino acid motifs characteristic of fibroin and consist of amino acids of about 100 residues.

The term "modified fibroin" in the present specification refers to artificially produced fibroin (artificial fibroin). The modified fibroin may be fibroin in which a domain sequence is different from an amino acid sequence of naturally derived fibroin or may be fibroin in which a domain sequence is the same as an amino acid sequence of naturally derived fibroin. The "naturally derived fibroin" referred to in the present specification is also a protein containing a domain sequence represented by Formula 1: [(A)$_n$ motif-REP]$_m$ or Formula 2: [(A)$_n$ motif-REP]$_m$- (A)$_n$ motif.

The "modified fibroin" may be fibroin obtained by using an amino acid sequence of naturally derived fibroin as it is, fibroin in which an amino acid sequence is modified based on an amino acid sequence of naturally derived fibroin (for example, fibroin in which an amino acid sequence is modified by modifying a cloned gene sequence of naturally derived fibroin), or fibroin artificially designed and synthesized independently of naturally derived fibroin (for example, fibroin having a desired amino acid sequence by chemically synthesizing a nucleic acid encoding a designed amino acid sequence).

In the present specification, the term "domain sequence" refers to an amino acid sequence which produces a crystalline region (typically, corresponding to an $(A)_n$ motif of an amino acid sequence) and an amorphous region (typically, corresponding to REP of an amino acid sequence) specific to fibroin, and refers to an amino acid sequence represented by Formula 1: $[(A)_n \text{ motif-REP}]_m$ or Formula 2: $[(A)_n \text{ motif -REP}]_m\text{- } (A)_n$ motif. Here, the $(A)_n$ motif represents an amino acid sequence mainly consisting of alanine residues, and the number of amino acid residues in the $(A)_n$ motif is 2 to 27. The number of amino acid residues in the $(A)_n$ motif may be an integer of 2 to 20, 4 to 27, 4 to 20, 8 to 20, 10 to 20, 4 to 16, 8 to 16, or 10 to 16. In addition, a proportion of the number of alanine residues to a total number of amino acid residues in the $(A)_n$ motif may be 40% or more, and may also be 60% or more, 70% or more, 80% or more, 83% or more, 85% or more, 86% or more, 90% or more, 95% or more, or 100% (which means that the $(A)_n$ motif consists of only alanine residues). At least a plurality of seven $(A)_n$ motifs present in the domain sequence may consist of only alanine residues. The REP represents an amino acid sequence consisting of 2 to 200 amino acid residues. The REP may be an amino acid sequence consisting of 10 to 200 amino acid residues or may be an amino acid sequence consisting of 10 to 40, 10 to 60, 10 to 80, 10 to 100, 10 to 120, 10 to 140, 10 to 160, or 10 to 180 amino acid residues. m represents an integer of 2 to 300, and may be an integer of 8 to 300, 10 to 300, 20 to 300, 40 to 300, 60 to 300, 80 to 300, 10 to 200, 20 to 200, 20 to 180, 20 to 160, 20 to 140, or 20 to 120. The plurality of $(A)_n$ motifs may be the same amino acid sequences or different amino acid sequences. A plurality of REP's may be the same amino acid sequences or different amino acid sequences.

The modified fibroin according to the present disclosure can be obtained by, for example, performing modification of an amino acid sequence corresponding to substitution, deletion, insertion, and/or addition of one or a plurality of amino acid residues with respect to a cloned gene sequence of naturally derived fibroin. Substitution, deletion, insertion, and/or addition of the amino acid residues can be performed by methods well known to those skilled in the art, such as site-directed mutagenesis. Specifically, it can be performed according to a method described in literatures such as Nucleic Acid Res. 10, 6487 (1982) and Methods in Enzymology, 100, 448 (1983).

The naturally derived fibroin is a protein containing a domain sequence represented by Formula 1: $[(A)_n \text{ motif-REP}]_m$ or Formula 2: $[(A)_n \text{ motif-REP}]_m\text{- } (A)_n$ motif, and a specific example thereof can include fibroin produced by insects or spiders.

Examples of the fibroin produced by insects can include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama, and hornet silk proteins discharged from larvae of Vespa simillima xanthoptera.

A more specific example of the fibroin produced by insects can include a silkworm fibroin L chain (GenBank Accession No. M76430 (base sequence) and AAA27840.1 (amino acid sequence)).

Examples of the fibroin produced by spiders can include spider silk proteins produced by spiders belonging to the genus Araneus such as Araneus ventricosus, Araneus diadematus, Araneus quadratus, Araneus pentagrammicus, and Araneus nojimai, spiders belonging to the genus Neoscona such as Neoscona scylla, Neoscona nautica, Neoscona adianta, and Neoscona scylloides, spiders belonging to the genus Pronus such as Pronouns minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta, and Argiope bruennichi, spiders belonging to the genus Arachnura such as Arachnura logio, spiders belonging to the genus Acusilas such as Acusilas coccineus, spiders belonging to the genus Cytophora such as Cyrtophora moluccensis, Cyrtophora exanthematica, and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata, and Cyclosa atrata, and spiders belonging to the genus Chorizopes such as Chorizopes nipponicus, and spider silk proteins produced by spiders belonging to the genus Tetragnatha such as Tetragnatha praedonia, Tetragnatha maxillosa, Tetragnatha extensa, and Tetragnatha squamata, spiders belonging to the genus Leucauge such as Leucauge magnifica, Leucauge blanda, and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus, and Latrodectus tredecimguttatus, and spiders belonging to the family Tetragnathidae such as spiders belonging to the genus Euprosthenops. Examples of the spider silk protein can include traction fiber proteins such as MaSp (MaSp1 and MaSp2) and ADF (ADF3 and ADF4), and MiSp (MiSp1 and MiSp2).

More specific examples of the spider silk protein produced by spiders can include fibroin-3 (adf-3) [derived from Araneus diadematus] (GenBank Accession No. AAC47010 (amino acid sequence), U47855 (base sequence)), fibroin-4 (adf-4) [derived from Araneus diadematus] (GenBank Accession No. AAC47011 (amino acid sequence), U47856 (base sequence)), dragline silk protein spidroin 1 [derived from Nephila clavipes] (GenBank Accession No. AAC04504 (amino acid sequence), U37520 (base sequence)), major ampullate spidroin 1 [derived from Latrodectus hesperus] (GenBank Accession No. ABR68856 (amino acid sequence), EF595246 (base sequence)), dragline silk protein spidroin 2 [derived from Nephila clavata] (GenBank Accession No. AAL32472 (amino acid sequence), AF441245 (base sequence)), major ampullate spidroin 1 [derived from Euprosthenops australis] (GenBank Accession No. CAJ00428 (amino acid sequence), AJ973155 (base sequence)), major ampullate spidroin 2 [Euprosthenops australis] (GenBank Accession No. CAM32249.1 (amino acid sequence), AM490169 (base sequence)), minor ampullate silk protein 1 [Nephila clavipes] (GenBank Accession No. AAC14589.1 (amino acid sequence)), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

A still more specific example of the naturally derived fibroin can include fibroin with sequence information registered in NCBI GenBank. For example, it can be confirmed by extracting sequences in which spidroin, ampullate, fibroin, "silk and polypeptide", or "silk and protein" is described as a keyword in DEFINITION among sequences containing INV as DIVISION among sequence information registered in NCBI GenBank, sequences in which a specific character string of products is described from CDS, or sequences in which a specific character string is described from SOURCE to TISSUE TYPE.

The modified fibroin according to the present disclosure may be modified silk fibroin (in which an amino acid sequence of silk protein produced by silkworm is modified), may be modified spider silk fibroin (in which an amino acid sequence of a spider silk protein produced by spiders is modified), or may be sericin-removed silk fibroin (so-called regenerated silk fibroin). The sericin-removed silk fibroin is purified by removing sericin covering silk fibroin and other fat contents. Modified spider silk fibroin is preferred as the modified fibroin.

Specific examples of the modified fibroin can include modified fibroin derived from a major dragline silk protein produced in a major ampullate gland of a spider (first modified fibroin), modified fibroin containing a domain sequence in which a content of glycine residues is reduced (second modified fibroin), modified fibroin containing a domain sequence in which a content of an $(A)_n$ motif is reduced (third modified fibroin), modified fibroin containing a domain sequence in which a content of glycine residues and a content of an $(A)_n$ motif are reduced (fourth modified fibroin), modified fibroin containing a domain sequence including a region locally having a high hydropathy index (fifth modified fibroin), and modified fibroin containing a domain sequence in which a content of glutamine residues is reduced (sixth modified fibroin).

An example of the first modified fibroin can include a protein containing a domain sequence represented by Formula 1: $[(A)_n$ motif-REP$]_m$. In the first modified fibroin, the number of amino acid residues in the $(A)_n$ motif is preferably an integer of 3 to 20, more preferably an integer of 4 to 20, still more preferably an integer of 8 to 20, still more preferably an integer of 10 to 20, still more preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, and still more preferably 20 to 100 residues, and still more preferably 20 to 75 residues. In the first modified fibroin, a total number of glycine residues, serine residues, and alanine residues contained in the amino acid sequence represented by Formula 1: $[(A)_n$ motif-REP$]_m$ is preferably 40% or more, more preferably 60% or more, and still more preferably 70% or more, with respect to a total number of amino acid residues.

The first modified fibroin may be a polypeptide having an amino acid sequence unit represented by Formula 1: $[(A)_n$ motif-REP$]_m$, and having a C-terminal sequence which is an amino acid sequence set forth in any one of SEQ ID NOs: 1 to 3 or a C-terminal sequence which is an amino acid sequence having 90% or more homology with the amino acid sequence set forth in any one of SEQ ID NOs: 1 to 3.

The amino acid sequence set forth in SEQ ID NO: 1 is identical to an amino acid sequence consisting of 50 amino acid residues at the C-terminus of an amino acid sequence of ADF3 (GI:1263287, NCBI), the amino acid sequence set forth in SEQ ID NO: 2 is identical to an amino acid sequence obtained by removing 20 residues from the C-terminus of the amino acid sequence set forth in SEQ ID NO: 1, and the amino acid sequence set forth in SEQ ID NO: 3 is identical to an amino acid sequence obtained by removing 29 residues from the C-terminus of the amino acid sequence set forth in SEQ ID NO: 1.

A more specific example of the first modified fibroin can include modified fibroin having an amino acid sequence set forth in (1-i) SEQ ID NO: 4 (recombinant spider silk protein ADF3KaiLargeNRSH1), or (1-ii) an amino acid sequence having 90% or more sequence identity with the amino acid sequence set forth in (1-i) SEQ ID NO: 4. The sequence identity is preferably 95% or more.

The amino acid sequence set forth in SEQ ID NO: 4 is an amino acid sequence obtained by approximately doubling first to thirteenth repeating regions and performing mutation so that translation is terminated at the 1154th amino acid residue in an amino acid sequence obtained by adding the amino acid sequence (SEQ ID NO: 5) of ADF3 consisting of a start codon, a His10 tag, and a recognition site for HRV3C protease (human rhinovirus 3C protease) to the N-terminus thereof. The C-terminal amino acid sequence of the amino acid sequence set forth in SEQ ID NO: 4 is identical to the amino acid sequence set forth in SEQ ID NO: 3.

The modified fibroin of (1-i) may consist of the amino acid sequence set forth in SEQ ID NO: 4.

The domain sequence of the second modified fibroin has an amino acid sequence in which a content of glycine residues is reduced, as compared with the naturally derived fibroin. It can be said that the second modified fibroin has an amino acid sequence corresponding to an amino acid sequence in which at least one or a plurality of glycine residues in REP are substituted with another amino acid residue, as compared with the naturally derived fibroin.

The domain sequence of the second modified fibroin may have an amino acid sequence corresponding to an amino acid sequence in which one glycine residue in at least one or the plurality of motif sequences is substituted with another amino acid residue, in at least one motif sequence selected from GGX and GPGXX (where G represents a glycine residue, P represents a proline residue, and X represents an amino acid residue other than glycine) in REP, as compared with the naturally derived fibroin.

In the second modified fibroin, a proportion of the motif sequences in which the above-described glycine residue is substituted with another amino acid residue may be 10% or more with respect to the entire motif sequences.

The second modified fibroin may contain a domain sequence represented by Formula 1: $[(A)_n$ motif-REP$]_m$ and may have an amino acid sequence in which z/w is 30% or more, 40% or more, 50% or more, or 50.9% or more, in which a total number of amino acid residues in an amino acid sequence consisting of XGX (where X represents an amino acid residue other than glycine) contained in all REP's in a sequence excluding the sequence from the $(A)_n$ motif located at the most C-terminal side to the C-terminus of the domain sequence from the domain sequence is z, and a total number of amino acid residues in a sequence excluding the sequence from the $(A)_n$ motif located at the most C-terminal side to the C-terminus of the domain sequence from the domain sequence is w. The number of alanine residues with respect to the total number of amino acid residues in the $(A)_n$ motif is 83% or more, preferably 86% or more, more preferably 90% or more, still more preferably 95% or more, and still more preferably 100% (which means that the $(A)_n$ motif consists of only alanine residues).

In the second modified fibroin, a content ratio of the amino acid sequence consisting of XGX is preferably increased by substituting one glycine residue in a GGX motif with another amino acid residue. In the second modified fibroin, a content ratio of an amino acid sequence consisting of GGX in the domain sequence is preferably 30% or less, more preferably 20% or less, still more preferably 10% or less, still more preferably 6% or less, still more preferably 4% or less, and particularly preferably 2% or less. The content ratio of the amino acid sequence consisting of GGX in the domain sequence can be calculated by the same method as the following calculation method of a content ratio (z/w) of the amino acid sequence consisting of XGX.

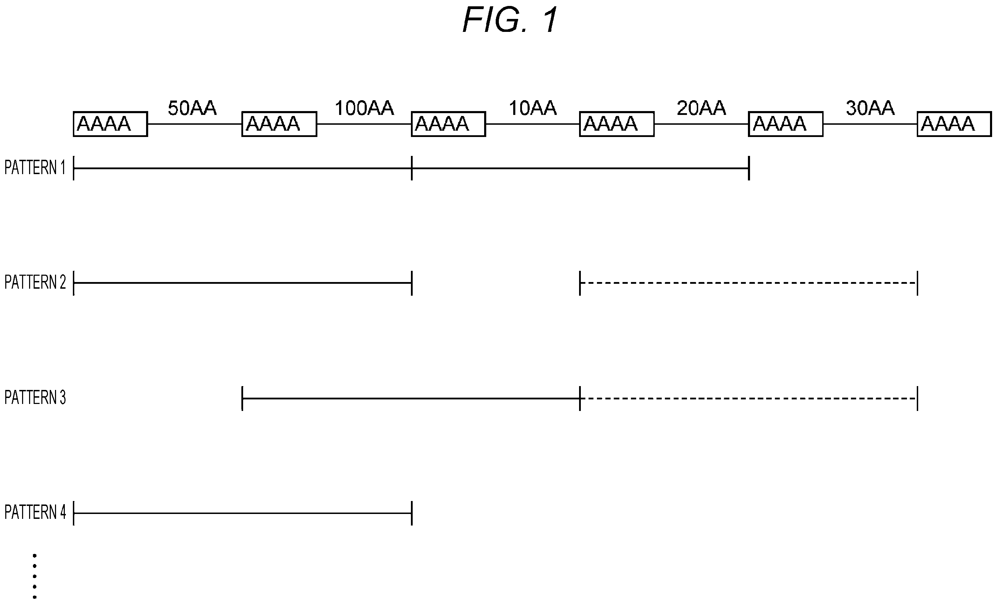

The calculation method of z/w will be described in more detail. First, the amino acid sequence consisting of XGX is extracted from all the REP's contained in the sequence excluding the sequence from the $(A)_n$ motif located at the most C-terminal side to the C-terminus of the domain sequence from the domain sequence in the fibroin containing the domain sequence represented by Formula 1: $[(A)_n$ motif-REP$]_m$ (modified fibroin or naturally derived fibroin). A total number of amino acid residues consisting of XGX is z. For example, in a case where 50 amino acid sequences consisting of XGX are extracted (there is no overlap), z is $50 \times 3 = 150$. In addition, for example, in a case where two Xs (central X) contained in XGX are present as in a case of an amino acid sequence consisting of XGXGX, it is calculated by subtracting the overlapping portion (in the case of XGXGX, z is 5 amino acid residues). w is a total number of amino acid residues contained in the sequence excluding the sequence from the $(A)_n$ motif located at the most C-terminal side to the C-terminus of the domain sequence from the domain sequence. For example, in the case of the domain sequence illustrated in Fig. 1, w is $4 + 50 + 4 + 100 + 4 + 10 + 4 + 20 + 4 + 30 = 230$ (excluding the $(A)_n$ motif located at the most C-terminal side). Next, z/w (%) can be calculated by dividing z by w.

Here, z/w in the naturally derived fibroin will be described. First, as described above, 663 types of fibroins (415 types of fibroins derived from spiders among them) were extracted by confirming fibroins with amino acid sequence information registered in NCBI GenBank by an exemplified method. z/w was calculated by the above-described calculation method from the amino acid sequences of the naturally derived fibroins which contain a domain sequence represented by Formula 1: $[(A)_n$ motif-REP$]_m$ and in which the content ratio of the amino acid sequence consisting of GGX in the fibroin is 6% or less, among all the extracted fibroins. As a result, z/w in each of the naturally derived fibroins is less than 50.9% (highest, 50.86%).

In the second modified fibroin, z/w is preferably 50.9% or more, more preferably 56.1% or more, still more preferably 58.7% or more, still more preferably 70% or more, and still more preferably 80% or more. An upper limit of z/w is not particularly limited, but may be, for example, 95% or less.

The second modified fibroin can be obtained by, for example, substituting and modifying at least a part of a base sequence encoding a glycine residue from a cloned gene sequence of naturally derived fibroin so as to encode another amino acid residue. In this case, one glycine residue in a GGX motif or a GPGXX motif may be selected as the glycine residue to be modified, and substitution may be performed so that z/w is 50.9% or more. In addition, the second modified fibroin can also be obtained by, for example, designing an amino acid sequence satisfying each of the above aspects from the amino acid sequence of the naturally derived fibroin, and chemically synthesizing a nucleic acid encoding the designed amino acid sequence. In any case, in addition to the modification corresponding to substitution of a glycine residue in the REP with another amino acid residue from the amino acid sequence of the naturally derived fibroin, modification of the amino acid sequence corresponding to substitution, deletion, insertion, and/or addition of one or a plurality of amino acid residues may be performed.

The above-described another amino acid residue is not particularly limited as long as it is an amino acid residue other than a glycine residue, but it is preferably a hydrophobic amino acid residue such as a valine (V) residue, a leucine (L) residue, an isoleucine (I) residue, a methionine (M) residue, a proline (P) residue, a phenylalanine (F) residue, or a tryptophan (W) residue, or a hydrophilic amino acid residue such as a glutamine (Q) residue, an asparagine (N) residue, a serine (S) residue, a lysine (K) residue, or a glutamic acid (E) residue, more preferably a valine (V) residue, a leucine (L) residue, an isoleucine (I) residue, a phenylalanine (F) residue, or a glutamine (Q) residue, and still more preferably a glutamine (Q) residue.

A more specific example of the second modified fibroin can include a modified fibroin having (2-i) an amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin of (2-i) will be described. The amino acid sequence set forth in SEQ ID NO: 6 is obtained by substituting GQX for all GGXs in REP of the amino acid sequence set forth in SEQ ID NO: 10 (Met-PRT313) corresponding to the naturally derived fibroin. The amino acid sequence set forth in SEQ ID NO: 7 is obtained by deleting every other two $(A)_n$ motifs from the N-terminus to the C-terminus from the amino acid sequence set forth in SEQ ID NO: 6 and further inserting one $[(A)_n$ motif-REP] before the C-terminal sequence. The amino acid sequence set forth in SEQ ID NO: 8 is obtained by inserting two alanine residues at the C-terminus of each $(A)_n$ motif of the amino acid sequence set forth in SEQ ID NO: 7 and further substituting a part of glutamine (Q) residues with a serine (S) residue to delete a part of amino acids at the C-terminus so as to be almost the same as a molecular weight of SEQ ID NO: 7. The amino acid sequence set forth in SEQ ID NO: 9 is an amino acid sequence obtained by adding a predetermined hinge sequence and a His tag sequence to the C-terminus of a sequence obtained by repeating a region of 20 domain sequences (where several amino acid residues on the C-terminal side of the region are substituted) present in the amino acid sequence set forth in SEQ ID NO: 7 four times.

A value of z/w in the amino acid sequence set forth in SEQ ID NO: 10 (corresponding to naturally derived fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y in the amino acid sequences set forth in SEQ ID NO: 10, SEQ ID NO: 6, SEQ ID NO: 7, SEQ ID NO: 8, and SEQ ID NO: 9 at a Giza ratio (described below) of 1:1.8 to 11.3 are 15.0%, 15.0%, 93.4%, 92.7%, and 89.8%, respectively.

The modified fibroin of (2-i) may consist of the amino acid sequence set forth in SEQ ID NO: 6, SEQ ID NO: 7, SEQ ID NO: 8, or SEQ ID NO: 9.

The modified fibroin of (2-ii) may consist of an amino acid sequence having 90% or more sequence identity with the amino acid sequence set forth in SEQ ID NO: 6, SEQ ID NO: 7, SEQ ID NO: 8, or SEQ ID NO: 9. The modified fibroin of (2-ii) is also a protein containing the domain sequence represented by Formula 1: $[(A)_n$ motif-REP]$_m$. The sequence identity is preferably 95% or more.

The modified fibroin of (2-ii) preferably has 90% or more sequence identity with the amino acid sequence set forth in SEQ ID NO: 6, SEQ ID NO: 7, SEQ ID NO: 8, or SEQ ID NO: 9, and z/w is preferably 50.9% or more, in which the total number of amino acid residues in the amino acid sequence consisting of XGX (where X represents the amino acid residue other than glycine) in the REP is z, and the total number of amino acid residues in the REP in the domain sequence is w.

The second modified fibroin may have a tag sequence at either or both of the N-terminus and the C-terminus. Therefore, it is possible to isolate, immobilize, detect, or visualize the modified fibroin.

An example of the tag sequence can include an affinity tag using specific affinity (binding property and affinity) with another molecule. A specific example of the affinity tag can include a histidine tag (His tag). The His tag is a short peptide in which about 4 to 10 histidine residues are arranged and has a property of specifically binding to a metal ion such as nickel. Thus, the His tag can be used for isolation of modified fibroin by chelating metal chromatography. A specific example of the tag sequence can include an amino acid sequence set forth in SEQ ID NO: 11 (amino acid sequence having a His tag sequence and a hinge sequence).

In addition, a tag sequence such as glutathione-S-transferase (GST) that specifically binds to glutathione or a maltose binding protein (MBP) that specifically binds to maltose can also be used.

Further, an "epitope tag" using an antigen-antibody reaction can also be used. By adding a peptide (epitope) showing antigenicity as a tag sequence, an antibody can be bound to the epitope. Examples of the epitope tag can include an HA (peptide sequence of hemagglutinin of influenza virus) tag, a myc tag, and a FLAG tag. The modified fibroin can be easily purified with high specificity by using the epitope tag.

Further, a tag sequence which can be cleaved with a specific protease can be used. By treating a protein adsorbed through the tag sequence with protease, it is also possible to recover the modified fibroin from which the tag sequence is cleaved.

A more specific example of the modified fibroin having a tag sequence can include modified fibroin having (2-iii) an amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

Each of amino acid sequences set forth in SEQ ID NO: 16 (PRT313), SEQ ID NO: 12, SEQ ID NO: 13, SEQ ID NO: 14, and SEQ ID NO: 15 is obtained by adding the amino acid sequence set forth in SEQ ID NO: 11 (having a His tag sequence and a hinge sequence) to the N-terminus of each of the amino acid sequences set forth in SEQ ID NO: 10, SEQ ID NO: 6, SEQ ID NO: 7, SEQ ID NO: 8, and SEQ ID NO: 9.

The modified fibroin of (2-iii) may consist of the amino acid sequence set forth in SEQ ID NO: 12, SEQ ID NO: 13, SEQ ID NO: 14, or SEQ ID NO: 15.

The modified fibroin of (2-iv) may consist of an amino acid sequence having 90% or more sequence identity with the amino acid sequence set forth in SEQ ID NO: 12, SEQ ID NO: 13, SEQ ID NO: 14, or SEQ ID NO: 15. The modified fibroin of (2-iv) is also a protein containing the domain sequence represented by Formula 1: $[(A)_n$ motif-REP$]_m$. The sequence identity is preferably 95% or more.

The modified fibroin of (2-iv) preferably has 90% or more sequence identity with the amino acid sequence set forth in SEQ ID NO: 12, SEQ ID NO: 13, SEQ ID NO: 14, or SEQ ID NO: 15, and z/w is preferably 50.9% or more, in which the total number of amino acid residues in the amino acid sequence consisting of XGX (where X represents the amino acid residue other than glycine) in the REP is z, and the total number of amino acid residues in the REP in the domain sequence is w.

The second modified fibroin may include a secretory signal for releasing the protein produced in a recombinant protein production system to the outside of a host. A sequence of the secretory signal can be appropriately set depending on a type of the host.

The domain sequence of the third modified fibroin has an amino acid sequence in which a content of an $(A)_n$ motif is reduced, as compared with the naturally derived fibroin. It can be said that the domain sequence of the third modified fibroin has an amino acid sequence corresponding to an amino acid sequence in which at least one or a plurality of $(A)_n$ motifs are deleted, as compared with the naturally derived fibroin.

The third modified fibroin may have an amino acid sequence corresponding to an amino acid sequence in which 10 to 40% of the $(A)_n$ motifs are deleted from the naturally derived fibroin.

The third modified fibroin may have an amino acid sequence corresponding to an amino acid sequence obtained by deleting one $(A)_n$ motif of every one to three $(A)_n$ motifs at least from the N-terminus to the C-terminus, as compared with the naturally derived fibroin.

The third modified fibroin may have an amino acid sequence corresponding to an amino acid sequence obtained by repeating deletion of at least two consecutive $(A)_n$ motifs and deletion of one $(A)_n$ motif in this order from the N-terminus to the C-terminus, as compared with the naturally derived fibroin.

The domain sequence of the third modified fibroin may have an amino acid sequence corresponding to an amino acid sequence obtained by deleting every other two $(A)_n$ motifs at least from the N-terminus to the C-terminus.

The third modified fibroin may contain a domain sequence represented by Formula 1: $[(A)_n$ motif-REP$]_m$, and may have an amino acid sequence in which x/y may be 20% or more, 30% or more, 40% or more, or 50% or more, in which when the number of amino acid residues in REP's in two $[(A)_n$ motif-REP] units adjacent to each other are sequentially compared from the N-terminus to the C-terminus, and then the number of amino acid residues in REP having a small number of amino acid residues is set as 1, a maximum value of the total value obtained by summing up the number of amino acid residues in the two adjacent $[(A)_n$ motif-REP] units where the ratio of the number of amino acid residues in the other REP is 1.8 to 11.3 is x, and the total number of amino acid residues in the domain sequence is y. The number of alanine residues with respect to the total number of amino acid residues in the $(A)_n$ motif is 83% or more, preferably 86% or more, more preferably 90% or more, still more preferably 95% or more, and still more preferably 100% (which means that the $(A)_n$ motif consists of only alanine residues).

The calculation method of x/y will be described in more detail with reference to Fig. 1. Fig. 1 illustrates a domain sequence excluding the N-terminal sequence and the C-terminal sequence from the modified fibroin. This domain sequence has a sequence of $(A)_n$ motif-first REP (50 amino acid residues) - $(A)_n$ motif-second REP (100 amino acid residues) - $(A)_n$ motif-third REP (10 amino acid residues) - $(A)_n$ motif-fourth REP (20 amino acid residues) - $(A)_n$ motif-fifth REP (30 amino acid residues) - $(A)_n$ motif from the N-terminal side (left side).

The two adjacent $[(A)_n$ motif-REP] units are sequentially selected from the N-terminus to the C-terminus so as not to overlap. In this case, an unselected $[(A)_n$ motif-REP] unit may exist. Fig. 1 illustrates a pattern 1 (a comparison between first REP and second REP and a comparison between third REP and fourth REP), a pattern 2 (a comparison between first REP and second REP and a comparison between fourth REP and fifth REP), a pattern 3 (a comparison between second REP and third REP and a comparison between fourth REP and fifth REP), and a pattern 4 (a comparison between first REP and second REP). There are selection methods other than this.

Next, for each pattern, the number of amino acid residues in each REP in the selected two adjacent [$(A)_n$ motif-REP] units is compared. The comparison is performed by determining a ratio of the number of amino acid residues in the other REP when one REP having a smaller number of amino acid residues is 1. For example, in the case of comparing the first REP (50 amino acid residues) with the second REP (100 amino acid residues), a ratio of the number of amino acid residues in the second REP when the first REP having a smaller number of amino acid residues is 1 is 100/50 = 2. Similarly, in the case of comparing the fourth REP (20 amino acid residues) with the fifth REP (30 amino acid residues), a ratio of the number of amino acid residues in the fifth REP when the fourth REP having a smaller number of amino acid residues is 1 is 30/20 = 1.5.

In Fig. 1, a set of [$(A)_n$ motif-REP] units in which the ratio of the number of amino acid residues in the other REP when one REP having a smaller number of amino acid residues is 1 is 1.8 to 11.3 is indicated by a solid line. In the present specification, the ratio is referred to as a Giza ratio. A set of [$(A)_n$ motif-REP] units in which the ratio of the number of amino acid residues in the other REP when one REP having a smaller number of amino acid residues is 1 is less than 1.8 or more than 11.3 is indicated by a broken line.

In each pattern, the number of all amino acid residues in two adjacent [$(A)_n$ motif-REP] units indicated by solid lines (including not only the number of amino acid residues in REP but also the number of amino acid residues in $(A)_n$ motif) are summed up. Then, the total values thus summed up are compared and the total value in the patterns at which the total value is maximized (the maximum value of the total value) is x. In the example illustrated in Fig. 1, the total value in the pattern 1 is the maximum.

Next, x/y (%) can be calculated by dividing x by the total amino acid residue number y of the domain sequence.

In the third modified fibroin, x/y is preferably 50% or more, more preferably 60% or more, still more preferably 65% or more, still more preferably 70% or more, still more preferably 75% or more, and particularly preferably 80% or more. An upper limit of x/y is not particularly limited, but may be, for example, 100% or less. In the case where the Giza ratio is 1:1.9 to 11.3, x/y is preferably 89.6% or more. In the case where the Giza ratio is 1:1.8 to 3.4, x/y is preferably 77.1% or more. In the case where the Giza ratio is 1:1.9 to 8.4, x/y is preferably 75.9% or more. In the case where the Giza ratio is 1:1.9 to 4.1, x/y is preferably 64.2% or more.

In the case where the third modified fibroin is modified fibroin in which at least a plurality of seven $(A)_n$ motifs present in the domain sequence consist of only alanine residues, x/y is preferably 46.4% or more, more preferably 50% or more, still more preferably 55% or more, still more preferably 60% or more, still more preferably 70% or more, and particularly preferably 80% or more. The upper limit of x/y is not particularly limited, but may be 100% or less.

Here, x/y in the naturally derived fibroin will be described. First, as described above, 663 types of fibroins (415 types of fibroins derived from spiders among them) were extracted by confirming fibroins with amino acid sequence information registered in NCBI GenBank by an exemplified method. x/y was calculated by the above-described calculation method from the amino acid sequences of naturally derived fibroins consisting of a domain sequence represented by Formula 1: [$(A)_n$ motif-REP]$_m$, among all the extracted fibroins. As a result, x/y in each of the naturally derived fibroins is less than 64.2% (highest, 64.14%).

The third modified fibroin can be obtained from, for example, a cloned gene sequence of naturally derived fibroin, by deleting one or a plurality of sequences encoding an $(A)_n$ motif so that x/y is 64.2% or more. In addition, for example, the third modified fibroin can also be obtained, from the amino acid sequence of naturally derived fibroin, by designing an amino acid sequence corresponding to deletion of one or a plurality of $(A)_n$ motifs so that x/y is 64.2% or more, and chemically synthesizing a nucleic acid encoding the designed amino acid sequence. In any case, in addition to the modification corresponding to deletion of the $(A)_n$ motif from the amino acid sequence of the naturally derived fibroin, modification of the amino acid sequence corresponding to substitution, deletion, insertion, and/or addition of one or a plurality of amino acid residues may be performed.

A more specific example of the third modified fibroin can include a modified fibroin having (3-i) an amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin of (3-i) will be described. The amino acid sequence set forth in SEQ ID NO: 17 is obtained by deleting every other two $(A)_n$ motifs from the N-terminus to the C-terminus from the amino acid sequence set forth in SEQ ID NO: 10 (Met-PRT313) corresponding to the naturally derived fibroin and further inserting one [$(A)_n$ motif-REP] before the C-terminal sequence. The amino acid sequence set forth in SEQ ID NO: 7, SEQ ID NO: 8, or SEQ ID NO: 9 is as described in the second modified fibroin.

The value of x/y in the amino acid sequence set forth in SEQ ID NO: 10 (corresponding to naturally derived fibroin) at a Giza ratio of 1:1.8 to 11.3 is 15.0%. Both the value of x/y in the amino acid sequence set forth in SEQ ID NO: 17 and the value of x/y in the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin of (3-i) may consist of the amino acid sequence set forth in SEQ ID NO: 17, SEQ ID NO: 7, SEQ ID NO: 8, or SEQ ID NO: 9.

The modified fibroin of (3-ii) may consist of an amino acid sequence having 90% or more sequence identity with the amino acid sequence set forth in SEQ ID NO: 17, SEQ ID NO: 7, SEQ ID NO: 8, or SEQ ID NO: 9. The modified fibroin of (3-ii) is also a protein containing the domain sequence represented by Formula 1: $[(A)_n$ motif-REP$]_m$. The sequence identity is preferably 95% or more.

The modified fibroin of (3-ii) preferably has 90% or more sequence identity with the amino acid sequence set forth in SEQ ID NO: 17, SEQ ID NO: 7, SEQ ID NO: 8, or SEQ ID NO: 9, and x/y is preferably 64.2% or more, in which when the number of amino acid residues in REP's in two $[(A)_n$ motif-REP$]$ units adjacent to each other are sequentially compared from the N-terminus to the C-terminus, and then the number of amino acid residues in REP having a small number of amino acid residues is set as 1, a maximum value of the total value obtained by summing up the number of amino acid residues in the two adjacent $[(A)_n$ motif-REP$]$ units where the ratio of the number of amino acid residues in the other REP is 1.8 to 11.3 (the Giza ratio is 1:1.8 to 11.3) is x, and the total number of amino acid residues in the domain sequence is y.

The third modified fibroin may have the above-described tag sequence at either or both of the N-terminus and the C-terminus.

A more specific example of the modified fibroin having a tag sequence can include modified fibroin having (3-iii) an amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

Each of the amino acid sequences set forth in SEQ ID NO: 18, SEQ ID NO: 13, SEQ ID NO: 14, and SEQ ID NO: 15 is obtained by adding the amino acid sequence set forth in SEQ ID NO: 11 (having a His tag sequence and a hinge sequence) to the N-terminus of each of the amino acid sequences set forth in SEQ ID NO: 17, SEQ ID NO: 7, SEQ ID NO: 8, and SEQ ID NO: 9.

The modified fibroin of (3-iii) may consist of the amino acid sequence set forth in SEQ ID NO: 18, SEQ ID NO: 13, SEQ ID NO: 14, or SEQ ID NO: 15.

The modified fibroin of (3-iv) may consist of an amino acid sequence having 90% or more sequence identity with the amino acid sequence set forth in SEQ ID NO: 18, SEQ ID NO: 13, SEQ ID NO: 14, or SEQ ID NO: 15. The modified fibroin of (3-iv) is also a protein containing the domain sequence represented by Formula 1: $[(A)_n$ motif-REP$]_m$. The sequence identity is preferably 95% or more.

The modified fibroin of (3-iv) preferably has 90% or more sequence identity with the amino acid sequence set forth in SEQ ID NO: 18, SEQ ID NO: 13, SEQ ID NO: 14, or SEQ ID NO: 15, and x/y is preferably 64.2% or more, in which when the number of amino acid residues in REP's in two $[(A)_n$ motif-REP$]$ units adjacent to each other are sequentially compared from the N-terminus to the C-terminus, and then the number of amino acid residues in REP having a small number of amino acid residues is set as 1, a maximum value of the total value obtained by summing up the number of amino acid residues in the two adjacent $[(A)_n$ motif-REP$]$ units where the ratio of the number of amino acid residues in the other REP is 1.8 to 11.3 is x, and the total number of amino acid residues in the domain sequence is y.

The third modified fibroin may include a secretory signal for releasing the protein produced in a recombinant protein production system to the outside of a host. A sequence of the secretory signal can be appropriately set depending on a type of the host.

The domain sequence of the fourth modified fibroin has an amino acid sequence in which a content of an $(A)_n$ motif and a content of glycine residues are reduced, as compared with the naturally derived fibroin. It can be said that the domain sequence of the fourth modified fibroin has an amino acid sequence corresponding to an amino acid sequence in which at least one or a plurality of $(A)_n$ motifs are deleted and at least one or a plurality of glycine residues in REP are substituted with another amino acid residue, as compared with the naturally derived fibroin. That is, the fourth modified fibroin is modified fibroin having the characteristics of the above-described second modified fibroin and third modified fibroin. Specific aspects and the like of the fourth modified fibroin are as described in the second modified fibroin and the third modified fibroin.

A more specific example of the fourth modified fibroin can include modified fibroin having (4-i) an amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific aspects of the modified fibroin having the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The domain sequence of the fifth modified fibroin may have an amino acid sequence including a region locally having a high hydropathy index corresponding to an amino acid sequence in which one or a plurality of amino acid residues in REP are substituted with amino acid residues having a high hydropathy index and/or one or a plurality of amino acid residues having a high hydropathy index are inserted into REP, as compared with the naturally derived fibroin.

It is preferable that the region locally having a high hydropathy index consists of two to four consecutive amino acid residues.

It is more preferable that the above-described amino acid residue having a high hydropathy index is an amino acid residue selected from isoleucine (I), valine (V), leucine (L), phenylalanine (F), cysteine (C), methionine (M), and alanine (A).

The fifth modified fibroin may be further subjected to modification of an amino acid sequence corresponding to substitution, deletion, insertion, and/or addition of one or a plurality of amino acid residues as compared with the naturally derived fibroin, in addition to modification corresponding to substitution of one or a plurality of amino acid residues in REP with amino acid residues having a high hydropathy index and/or insertion of one or a plurality of amino acid residues having a high hydropathy index into REP, as compared with the naturally derived fibroin.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) from a cloned gene sequence of naturally derived fibroin, and/or inserting one or a plurality of hydrophobic amino acid residues into REP. In addition, the fifth modified fibroin can be obtained by, for example, designing an amino acid sequence corresponding to substitution of one or a plurality of hydrophilic amino acid residues in REP with hydrophobic amino acid residues from an amino acid sequence of naturally derived fibroin, and/or insertion of one or a plurality of hydrophobic amino acid residues into REP, and chemically synthesizing a nucleic acid encoding the designed amino acid sequence. In any case, in addition to modification corresponding to substitution of one or a plurality of hydrophilic amino acid residues in REP with hydrophobic amino acid residues from amino acid sequences of naturally derived fibroin, and/or insertion of one or a plurality of hydrophobic amino acid residues into REP, modification of an amino acid sequence corresponding to substitution, deletion, insertion, and/or addition of one or a plurality of amino acid residues may be further performed.

The fifth modified fibroin may contain a domain sequence represented by Formula 1: $[(A)_n$ motif-REP$]_m$, and may have an amino acid sequence in which p/q is 6.2% or more, in which in all REP's contained in a sequence excluding a sequence from a $(A)_n$ motif located the most C-terminal side to the C-terminus of the domain sequence from the domain sequence, a total number of amino acid residues contained in a region where an average value of hydropathy indices of four consecutive amino acid residues is 2.6 or more is p, and a total number of amino acid residues contained in the sequence excluding the sequence from the $(A)_n$ motif located the most C-terminal side to the C-terminus of the domain sequence from the domain sequence is q.

A known index (Hydropathy index: Kyte J, & Doolittle R (1982), "A simple method for displaying the hydropathic character of a protein", J. Mol. Biol., 157, pp. 105-132) is used as the hydropathy index of the amino acid residue. Specifically, the hydropathy index (hereinafter, also referred to as "HI") of each amino acid is as shown in Table 1.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Asparaginic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

The calculation method of p/q will be described in more detail. In the calculation, the sequence excluding the sequence from the $(A)_n$ motif located at the most C-terminal side to the C-terminus of the domain sequence from the domain sequence represented by Formula 1: $[(A)_n$ motif-REP$]_m$ (hereinafter, referred to as "sequence A") is used. First, in all REP's contained in the sequence A, an average value of hydropathy indices of four consecutive amino acid residues is calculated. The average value of the hydropathy indices is determined by dividing the sum of HI of each of the amino acid residues contained in the four consecutive amino acid residues by 4 (the number of amino acid residues). The average value of the hydropathy indices is determined for all of the four consecutive amino acid residues (each of the amino acid residues is used for calculating the average value 1 to 4 times). Next, a region where the average value of the hydropathy indices of the four consecutive amino acid residues is 2.6 or more is specified. Even in a case where certain amino acid residues correspond to a plurality of "four consecutive amino acid residues having an average value of hydropathy indices of 2.6 or more", the amino acid residue is counted as one amino acid residue in the region. Then, the total number of amino acid residues contained in the region is p. In addition, the total number of amino acid residues contained in the sequence A is q.

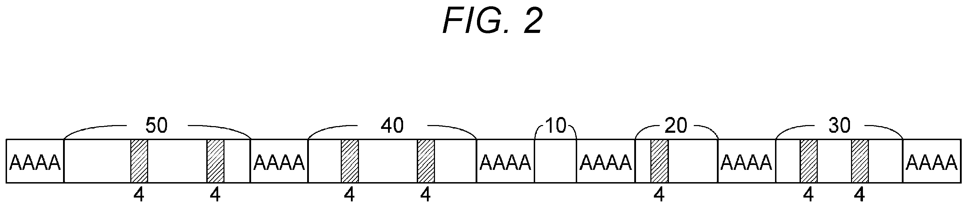

For example, in a case where the "four consecutive amino acid residues having an average value of the hydropathy indices of 2.6 or more" are extracted from 20 places (no overlap), in the region where the average value of the hydropathy indices of four consecutive amino acid residues is 2.6 or more, the number of the four consecutive amino acid residues (no overlap) is 20, and thus p is $20 \times 4 = 80$. In addition, for example, in a case where two of the "four consecutive amino acid residues having an average value of the hydropathy indices of 2.6 or more" overlap by only one amino acid residue, in the region where the average value of the hydropathy indices of four consecutive amino acid residues is 2.6 or more, the number of amino acid residues is 7 ($p = 2 \times 4 - 1 = 7$, "-1" is the deduction of overlap). For example, in the case of the domain sequence illustrated in Fig. 2, since the number of the "four consecutive amino acid residues having an average value of the hydropathy indices of 2.6 or more", which do not overlap, is 7, p is $7 \times 4 = 28$. In addition, for example, in the case of the domain sequence illustrated in Fig. 2, q is $4 + 50 + 4 + 40 + 4 + 10 + 4 + 20 + 4 + 30 = 170$ (excluding the $(A)_n$ motif located at the most C-terminal side). Next, p/q (%) can be calculated by dividing p by q. In the case of Fig. 2, 28/170 = 16.47%.

In the fifth modified fibroin, p/q is preferably 6.2% or more, more preferably 7% or more, still more preferably 10% or more, still more preferably 20% or more, and still more preferably 30% or more. An upper limit of p/q is not particularly limited, but may be, for example, 45% or less.

The fifth modified fibroin can be obtained by, for example, substituting one or a plurality of hydrophilic amino acid residues in REP (for example, amino acid residues having a negative hydropathy index) with hydrophobic amino acid residues (for example, amino acid residues having a positive hydropathy index) so that a cloned amino acid sequence of naturally derived fibroin satisfies the condition of p/q, and/or modifying the cloned amino acid sequence of naturally derived fibroin with an amino acid sequence including a region locally having a high hydropathy index by inserting one or a plurality of hydrophobic amino acid residues into REP. In addition, the fifth modified fibroin can also be obtained by, for example, designing an amino acid sequence satisfying the condition of p/q from the amino acid sequence of the naturally derived fibroin, and chemically synthesizing a nucleic acid encoding the designed amino acid sequence. In any case, modification corresponding to substitution, deletion, insertion, and/or addition of one or a plurality of amino acid residues may also be performed, in addition to modification corresponding to substitution of one or a plurality of amino acid residues in REP with amino acid residues having a high hydropathy index, and/or insertion of one or a plurality of amino acid residues having a high hydropathy index into REP, as compared with the naturally derived fibroin.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

A more specific example of the fifth modified fibroin can include modified fibroin having (5-i) an amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin of (5-i) will be described. The amino acid sequence set forth in SEQ ID NO: 19 is obtained by inserting an amino acid sequence consisting of three amino acid residues (VLI) at two sites for each REP into the amino acid sequence set forth in SEQ ID NO: 7 (Met-PRT410), except for the domain sequence at the end on the C-terminal side, and further substituting a part of glutamine (Q) residues with serine (S) residues and deleting a part of amino acids on the C-terminal side. The amino acid sequence set forth in SEQ ID NO: 20 is obtained by inserting the amino acid sequence consisting of three amino acid residues (VLI) at one site for each REP into the amino acid sequence set forth in SEQ ID NO: 8 (Met-PRT525). The amino acid sequence set forth in SEQ ID NO: 21 is obtained by inserting the amino acid sequence consisting of three amino acid residues (VLI) at two sites for each REP into the amino acid sequence set forth in SEQ ID NO: 8.

The modified fibroin of (5-i) may consist of the amino acid sequence set forth in SEQ ID NO: 19, SEQ ID NO: 20, or SEQ ID NO: 21.

The modified fibroin of (5-ii) may consist of an amino acid sequence having 90% or more sequence identity with the amino acid sequence set forth in SEQ ID NO: 19, SEQ ID NO: 20, or SEQ ID NO: 21. The modified fibroin of (5-ii) is also a protein containing the domain sequence represented by Formula **1**: $[(A)_n \text{ motif-REP}]_m$. The sequence identity is preferably 95% or more.

The modified fibroin of (5-ii) preferably has 90% or more sequence identity with the amino acid sequence set forth in SEQ ID NO: 19, SEQ ID NO: 20, or SEQ ID NO: 21, and p/q is preferably 6.2% or more, in which in all REP's contained in a sequence excluding a sequence from a $(A)_n$ motif located the most C-terminal side to the C-terminus of the domain sequence from the domain sequence, a total number of amino acid residues contained in a region where an average value of hydropathy indices of four consecutive amino acid residues is 2.6 or more is p, and a total number of amino acid residues contained in the sequence excluding the sequence from the $(A)_n$ motif located the most C-terminal side to the C-terminus of the domain sequence from the domain sequence is q.

The fifth modified fibroin may have a tag sequence at either or both of the N-terminus and the C-terminus.

A more specific example of the modified fibroin having a tag sequence can include modified fibroin having (5-iii) an amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

Each of the amino acid sequences set forth in SEQ ID NO: 22, SEQ ID NO: 23, and SEQ ID NO: 24 is obtained by adding the amino acid sequence set forth in SEQ ID NO: 11 (having a His tag sequence and a hinge sequence) to the N-terminus of each of the amino acid sequences set forth in SEQ ID NO: 19, SEQ ID NO: 20, and SEQ ID NO: 21.

The modified fibroin of (5-iii) may consist of the amino acid sequence set forth in SEQ ID NO: 22, SEQ ID NO: 23, or SEQ ID NO: 24.

The modified fibroin of (5-iv) may consist of an amino acid sequence having 90% or more sequence identity with the amino acid sequence set forth in SEQ ID NO: 22, SEQ ID NO: 23, or SEQ ID NO: 24. The modified fibroin of (5-iv) is also a protein containing the domain sequence represented by Formula **1:** $[(A)_n \text{ motif-REP}]_m$. The sequence identity is preferably 95% or more.

The modified fibroin of (5-iv) preferably has 90% or more sequence identity with the amino acid sequence set forth in SEQ ID NO: 22, SEQ ID NO: 23, or SEQ ID NO: 24, and p/q is preferably 6.2% or more, in which in all REP's contained in a sequence excluding a sequence from a $(A)_n$ motif located the most C-terminal side to the C-terminus of the domain sequence from the domain sequence, a total number of amino acid residues contained in a region where an average value of hydropathy indices of four consecutive amino acid residues is 2.6 or more is p, and a total number of amino acid residues contained in the sequence excluding the sequence from the $(A)_n$ motif located the most C-terminal side to the C-terminus of the domain sequence from the domain sequence is **q.**

The fifth modified fibroin may include a secretory signal for releasing the protein produced in a recombinant protein production system to the outside of a host. A sequence of the secretory signal can be appropriately set depending on a type of the host.

The sixth modified fibroin has an amino acid sequence in which a content of glutamine residues is reduced, as compared with the naturally derived fibroin.

The sixth modified fibroin preferably contains at least one motif selected from a GGX motif and a GPGXX motif in the amino acid sequence of REP.

In a case where the sixth modified fibroin contains the GPGXX motif in REP, a content rate of the GPGXX motif is generally 1% or more, may be 5% or more, and is preferably 10% or more. An upper limit of the content rate of the GPGXX motif is not particularly limited, but may be 50% or less or 30% or less.

In the present specification, the "content rate of the GPGXX motif" is a value calculated by the following method.

In fibroin (modified fibroin or naturally derived fibroin) containing a domain sequence represented by Formula 1: $[(A)_n \text{ motif -REP}]_m$ or Formula 2: $[(A)_n \text{ motif-REP}]_m - (A)_n$ motif, the content rate of the GPGXX motif is calculated as s/t, in which the number obtained by tripling the total number of GPGXX motifs in the regions of all REP's contained in a sequence excluding the sequence from the $(A)_n$ motif located at the most C-terminal side to the C-terminus of the domain sequence from the domain sequence (that is, corresponding to the total number of G and P in the GPGXX motifs) is s, and the total number of amino acid residues in all REP's excluding the sequence from the $(A)_n$ motif located at the most C-terminal side to the C-terminus of the domain sequence from the domain sequence and further excluding the $(A)_n$ motifs is t.

For the calculation of the content rate of the GPGXX motif, the "sequence excluding a sequence from the $(A)_n$ motif located at the most C-terminal side to the C-terminus of the domain sequence from the domain sequence" is used to exclude the effect occurring due to the fact that the "sequence from the $(A)_n$ motif located at the most C-terminal side to the C-terminus of the domain sequence" (sequence corresponding to REP) may have a sequence having a low correlation with the sequence characteristic of fibroin, which influences the calculation result of the content rate of the GPGXX motif in a case where m is small (that is, in a case where the domain sequence is short). In a case where the "GPGXX motif" is located at the C-terminus of REP, it is regarded as the "GPGXX motif" even in a case where "XX" is, for example, "AA".

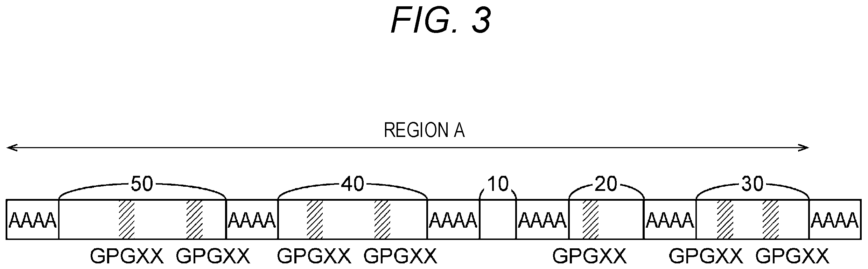

Fig. 3 is a schematic diagram illustrating a domain sequence of modified fibroin. The calculation method of the content rate of the GPGXX motif will be specifically described with reference to Fig. 3. First, in the domain sequence of the modified fibroin ("$[(A)_n \text{ motif-REP}]_m - (A)_n$ motif" type) illustrated in Fig. 3, since all REP's are contained in the "sequence excluding the sequence from the $(A)_n$ motif located at the most C-terminal side to the C-terminus of the domain sequence from the domain sequence" (the sequence indicated by the "region A" in Fig. 3), the number of GPGXX motifs for calculating s is 7, and s is $7 \times 3 = 21$. Similarly, since all REP's are contained in the "sequence excluding the sequence from the $(A)_n$ motif located at the most C-terminal side to the C-terminus of the domain sequence from the domain sequence" (the sequence indicated by the "region A" in Fig. 3), a total number t of amino acid residues in all REP's further excluding

$(A)_n$ motifs from the sequence is 50 + 40 + 10 + 20 + 30 = 150. Next, s/t (%) can be calculated by dividing s by t, and in the case of the modified fibroin of Fig. 3, s/t (%) is 21/150 = 14.0%.

In the sixth modified fibroin, a content rate of glutamine residues is preferably 9% or less, more preferably 7% or less, still more preferably 4% or less, and particularly preferably 0%.

In the present specification, the "content rate of the glutamine residues" is a value calculated by the following method.

In fibroin (modified fibroin or naturally derived fibroin) containing a domain sequence represented by Formula 1: $[(A)_n$ motif -REP$]_m$ or Formula 2: $[(A)_n$ motif-REP$]_m$- $(A)_n$ motif, the content rate of the glutamine residues is calculated as u/t, in which a total number of glutamine residues in regions of all REP's contained in a sequence excluding the sequence from the $(A)_n$ motif located at the most C-terminal side to the C-terminus of the domain sequence from the domain sequence (sequence corresponding to the "region A" in Fig. 3) is u, and a total number of amino acid residues in all REP's excluding the sequence from the $(A)_n$ motif located at the most C-terminal side to the C-terminus of the domain sequence from the domain sequence and further excluding $(A)_n$ motifs is t. For the calculation of the content rate of the glutamine residues, the "sequence excluding the sequence from the $(A)_n$ motif located at the most C-terminal side to the C-terminus of the domain sequence from the domain sequence" is used for the same reason described above.

The domain sequence of the sixth modified fibroin may have an amino acid sequence corresponding to deletion of one or a plurality of glutamine residues in REP, or substitution of one or a plurality of glutamine residues with another amino acid residue, as compared with the naturally derived fibroin.

"Another amino acid residue" may be an amino acid residue other than a glutamine residue, but is preferably an amino acid residue having a higher hydropathy index than that of a glutamine residue. The hydropathy index of the amino acid residue is shown in Table **1**.

As shown in Table 1, examples of the amino acid residue having a higher hydropathy index than that of a glutamine residue can include an amino acid residue selected from isoleucine (I), valine (V), leucine (L), phenylalanine (F), cysteine (C), methionine (M) alanine (A), glycine (G), threonine (T), serine (S), tryptophan (W), tyrosine (Y), proline (P), and histidine (H). Among them, an amino acid residue selected from isoleucine (I), valine (V), leucine (L), phenylalanine (F), cysteine (C), methionine (M), and alanine (A) is more preferred, and an amino acid residue selected from isoleucine (I), valine (V), leucine (L), and phenylalanine (F) is still more preferred.

In the sixth modified fibroin, hydrophobicity of REP is preferably -0.8 or more, more preferably -0.7 or more, still more preferably 0 or more, still more preferably 0.3 or more, and still more preferably 0.4 or more. An upper limit of the hydrophobicity of REP is not particularly limited, but may be 1.0 or less or 0.7 or less.

In the present specification, the "hydrophobicity of REP" is a value calculated by the following method.

In fibroin (modified fibroin or naturally derived fibroin) containing a domain sequence represented by Formula 1: $[(A)_n$ motif -REP$]_m$ or Formula 2: $[(A)_n$ motif-REP$]_m$- $(A)_n$ motif, the hydrophobicity of REP is calculated as v/t, in which the sum of hydropathy indices of the amino acid residues in the regions of all REP's contained in the sequence excluding the sequence from the $(A)_n$ motif located at the most C-terminal side to the C-terminus of the domain sequence from the domain sequence (sequence corresponding to the "region A" in Fig. 3) is v, and the total number of amino acid residues in all REP's excluding the sequence from the $(A)_n$ motif located at the most C-terminal side to the C-terminus of the domain sequence from the domain sequence and further excluding $(A)_n$ motifs is t. For the calculation of the hydrophobicity of REP, the "sequence excluding the sequence from the $(A)_n$ motif located at the most C-terminal side to the C-terminus of the domain sequence from the domain sequence" is used for the same reason described above.

The sixth modified fibroin may be further subjected to modification of an amino acid sequence corresponding to substitution, deletion, insertion, and/or addition of one or a plurality of amino acid residues, in addition to modification corresponding to deletion of one or a plurality of glutamine residues in REP, and/or substitution of one or a plurality of glutamine residues in REP with another amino acid residue, as compared to naturally derived fibroin.

The sixth modified fibroin can be obtained by, for example, deleting one or a plurality of glutamine residues in REP from a cloned gene sequence of naturally derived fibroin, and/or substituting one or a plurality of glutamine residues in REP with another amino acid residue. In addition, the sixth modified fibroin can be obtained by, for example, designing an amino acid sequence corresponding to deletion of one or a plurality of glutamine residues in REP from an amino acid sequence of naturally derived fibroin, and/or substitution of one or a plurality of glutamine residues in REP with another amino acid residue, and chemically synthesizing a nucleic acid encoding the designed amino acid sequence.

More specific examples of the sixth modified fibroin can include modified fibroin having (6-i) an amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), SEQ ID NO: 32 (Met-PRT966), SEQ ID NO: 41 (Met-PRT917), or SEQ ID NO: 42 (Met-PRT1028), and modified fibroin having (6-ii) an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-i) will be described. The amino acid sequence set forth in SEQ ID NO: 25 is obtained by substituting all QQs in the amino acid sequence set forth in SEQ ID NO: 7 (Met-PRT410) with VL. The amino acid sequence set forth in SEQ ID NO: 26 is obtained by substituting all QQs in the amino acid sequence set forth in SEQ ID NO: 7 with TS and substituting the remaining Q with A. The amino acid sequence set forth in SEQ ID NO: 27 is obtained by substituting all QQs in the amino acid sequence set forth in SEQ ID NO: 7 with VL and substituting the remaining Q with I. The amino acid sequence set forth in SEQ ID NO: 28 is obtained by substituting all QQs in the amino acid sequence set forth in SEQ ID NO: 7 with VI and substituting the remaining Q with L. The amino acid sequence set forth in SEQ ID NO: 29 is obtained by substituting all QQs in the amino acid sequence set forth in SEQ ID NO: 7 with VF and substituting the remaining Q with I.

The amino acid sequence set forth in SEQ ID NO: 30 is obtained by substituting all QQs in the amino acid sequence set forth in SEQ ID NO: 8 (Met-PRT525) with VL. The amino acid sequence set forth in SEQ ID NO: 31 is obtained by substituting all QQs in the amino acid sequence set forth in SEQ ID NO: 8 with VL and substituting the remaining Q with I.

The amino acid sequence set forth in SEQ ID NO: 32 is obtained by substituting, with VF, all QQs in a sequence obtained by repeating a region of 20 domain sequences present in the amino acid sequence set forth in SEQ ID NO: 7 (Met-PRT410) two times and substituting the remaining Q with I.

The amino acid sequence set forth in SEQ ID NO: 41 (Met-PRT917) is obtained by substituting all QQs in the amino acid sequence set forth in SEQ ID NO: 7 with LI and substituting the remaining Q with V. The amino acid sequence set forth in SEQ ID NO: 42 (Met-PRT1028) is obtained by substituting all QQs in the amino acid sequence set forth in SEQ ID NO: 7 with IF and substituting the remaining Q with T.

The content rate of the glutamine residues in each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42 is 9% or less (Table 2).

[Table 2]

| Modified fibroin | Content of glutamine residue | Content of GPGXX motif | Hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |
| Met-PRT966 (SEQ ID NO: 32) | 0.0% | 28.0% | 0.35 |
| Met-PRT917 (SEQ ID NO: 41) | 0.0% | 27.9% | 0.46 |
| Met-PRT1028 (SEQ ID NO: 42) | 0.0% | 28.1% | 0.05 |

The modified fibroin of (6-i) may consist of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42.

The modified fibroin of (6-ii) may consist of the amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, or SEQ ID NO: 42. The modified fibroin of (6-ii) is also a protein containing a domain sequence represented by Formula 1: $[(A)_n \text{ motif-REP}]_m$ or Formula 2: $[(A)_n \text{ motif-REP}]_m\text{-}(A)_n$ motif. The sequence identity is preferably 95% or more.

It is preferable that a content rate of glutamine residues in the modified fibroin of (6-ii) is preferably 9% or less. In addition, it is preferable that a content rate of a GPGXX motif in the modified fibroin of (6-ii) is preferably 10% or more.

The sixth modified fibroin may have a tag sequence at either or both of the N-terminus and the C-terminus. Therefore, it is possible to isolate, immobilize, detect, or visualize the modified fibroin.

More specific examples of the modified fibroin having a tag sequence can include modified fibroin having (6-iii) an amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37 (PRT918), SEQ ID NO: 38 (PRT699), SEQ ID NO: 39 (PRT698), SEQ ID NO: 40 (PRT966), SEQ ID NO: 43 (PRT917), or SEQ ID NO: 44 (PRT1028), or modified fibroin having (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

Each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, and SEQ ID NO: 44 is obtained by adding the amino acid sequence set forth in SEQ ID NO: 11 (having a His tag sequence and a hinge sequence) to the N-terminus of each of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 41, and SEQ ID NO: 42. Since only the tag sequence is added to the N-terminus, the content rate of the glutamine residues is not changed, and the content rate of the glutamine residues in each of the amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44 is 9% or less (Table 3).

[Table 3]

| Modified fibroin | Content of glutamine residue | Content of GPGXX motif | Hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0. 0% | 26.4% | -0.03 |
| PRT966 (SEQ ID NO: 40) | 0.0% | 28.0% | 0.35 |
| PRT917 (SEQ ID NO: 43) | 0.0% | 27.9% | 0.46 |
| PRT1028 (SEQ ID NO: 44) | 0.0% | 28.1% | 0.05 |

The modified fibroin of (6-iii) may consist of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44.

The modified fibroin of (6-iv) may consist of the amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 43, or SEQ ID NO: 44. The modified fibroin of (6-iv) is also a protein containing a domain sequence represented by Formula 1: $[(A)_n$ motif-$REP]_m$ or Formula 2: $[(A)_n$ motif-$REP]_m$-$(A)_n$ motif. The sequence identity is preferably 95% or more.

It is preferable that a content rate of glutamine residues in the modified fibroin of (6-iv) is preferably 9% or less. In addition, it is preferable that a content rate of a GPGXX motif in the modified fibroin of (6-iv) is preferably 10% or more.

The sixth modified fibroin may include a secretory signal for releasing the protein produced in a recombinant protein production system to the outside of a host. A sequence of the secretory signal can be appropriately set depending on a type of the host.

The modified fibroin may be modified fibroin having at least two or more characteristics of the characteristics of the first modified fibroin, the second modified fibroin, the third modified fibroin, the fourth modified fibroin, the fifth modified fibroin, and the sixth modified fibroin.

The modified fibroin may be hydrophilic modified fibroin or hydrophobic modified fibroin. The hydrophobic modified fibroin is modified fibroin in which a value obtained by determining the sum of hydropathy indices (HI) of all amino acid residues constituting the modified fibroin and then dividing the sum by the number of all amino acid residues (average HI) is 0 or more. The hydropathy index is as shown in Table **1.** In addition, the hydrophilic modified fibroin is modified fibroin in which the average HI is less than 0.

An example of the hydrophobic modified fibroin can include the above-described sixth modified fibroin. A more specific example of the hydrophobic modified fibroin can include modified fibroin having an amino acid sequence set forth in SEQ ID NO: 27, SEQ ID NO: 28, SEQ ID NO: 29, SEQ ID NO: 30, SEQ ID NO: 31, SEQ ID NO: 32, SEQ ID NO: 33, or SEQ ID NO: 43, or an amino acid sequence set forth in SEQ ID NO: 35, SEQ ID NO: 37, SEQ ID NO: 38, SEQ ID NO: 39, SEQ ID NO: 40, SEQ ID NO: 41, or SEQ ID NO: 44.

Examples of the hydrophilic modified fibroin can include the above-described first modified fibroin, second modified fibroin, third modified fibroin, fourth modified fibroin, and fifth modified fibroin. A more specific example of the hydrophilic modified fibroin can include fibroin having an amino acid sequence set forth in SEQ ID NO: 4, an amino acid sequence set forth in SEQ ID NO: 6, SEQ ID NO: 7, SEQ ID NO: 8, or SEQ ID NO: 9, an amino acid sequence set forth in SEQ ID NO: 13, SEQ ID NO: 11, SEQ ID NO: 14, or SEQ ID NO: 15, an amino acid sequence set forth in SEQ ID NO: 18, SEQ ID NO: 7, SEQ ID NO: 8, or SEQ ID NO: 9, an amino acid sequence set forth in SEQ ID NO: 17, SEQ ID NO: 11, SEQ ID NO: 14, or SEQ ID NO: 15, or an amino acid sequence set forth in SEQ ID NO: 19, SEQ ID NO: 20, or SEQ ID NO: 21.

(Production method of protein)

The protein can be produced by, for example, expressing a nucleic acid by a nucleic acid sequence encoding the protein and a host transformed with an expression vector having one or a plurality of regulatory sequences operably linked to the nucleic acid sequence.

A production method of a gene encoding the protein is not particularly limited. For example, the gene can be produced by a method in which a gene encoding a natural structural protein is amplified and cloned by a polymerase chain reaction (PCR) or the like, or a method of chemically synthesizing a gene. A method for chemically synthesizing a gene is not particularly limited. For example, genes can be chemically synthesized by a method of linking, by PCR or the like, oligonucleotides that are automatically synthesized by AKTA oligopilot plus 10/100 (GE Healthcare Japan Ltd.) or the like, based on the amino acid sequence information of the structural protein obtained from the web database of NCBI and the like. In this case, in order to facilitate purification or confirmation of the protein, a gene encoding a protein consisting of an amino acid sequence obtained by adding an amino acid sequence consisting of a start codon and a His10 tag to the N-terminus of the above amino acid sequence may be synthesized.

The regulatory sequence is a sequence that controls the expression of a protein in a host (for example, a promoter, an enhancer, a ribosome binding sequence, a transcription termination sequence, or the like), and can be appropriately selected depending on the type of the host. As a promoter, an inducible promoter which functions in host cells and is capable of inducing expression of a protein may be used. An inducible promoter is a promoter that can control transcription due to the presence of an inducer (expression inducer), the absence of a repressor molecule, or a physical factor such as an increase or decrease in temperature, osmotic pressure, or pH value.

The type of the expression vector such as a plasmid vector, a viral vector, a cosmid vector, a fosmid vector, or an artificial chromosome vector can be appropriately selected depending on the type of the host. As the expression vector, an expression vector which can automatically replicate in a host cell or can be incorporated into a chromosome of a host and which contains a promoter at a position capable of transcribing the nucleic acid encoding the protein is suitably used.

Both a prokaryote and a eukaryote such as yeast, filamentous fungi, insect cells, animal cells, and plant cells can be suitably used as hosts.

Preferred examples of the prokaryote can include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. An example of microorganisms belonging to the genus Escherichia can include **E. coli**. An example of microorganisms belonging to the genus Brevibacillus can include Brevibacillus agri. An example of microorganisms belonging to the genus Serratia can include Serratia liquefaciens. An example of microorganisms belonging to the genus Bacillus can include Bacillus subtilis. An example of microorganisms belonging to the genus Microbacterium can include Microbacterium ammoniaphilum. An example of microorganisms belonging to the genus Brevibacterium can include Brevibacterium divaricatum. An example of microorganisms belonging to the genus Corynebacterium can include Corynebacterium ammoniagenes. An example of microorganisms belonging to the genus Pseudomonas can include Pseudomonas putida.

In a case where a prokaryote is used as a host, examples of a vector into which a nucleic acid encoding the recombinant protein is introduced can include pBTrp2 (manufactured by Boehringer Mannheim GmbH), pGEX (manufactured by Pharmacia Corporation), and pUC18, pBluescriptII, pSupex, pET22b, pCold, pUB110, and pNCO2 (JP 2002-238569 A).

Examples of the eukaryotic host can include yeast and filamentous fungi (mold or the like). An example of the yeast can include yeast which belongs to the genus Saccharomyces, the genus Pichia, or the genus Schizosaccharomyces. An example of filamentous fungi can include filamentous fungi belonging to the genus Aspergillus, the genus Penicillium, or the genus Trichoderma.

In a case where a eukaryote is used as a host, examples of a vector into which a nucleic acid encoding the protein is introduced can include YEP13 (ATCC37115) and YEp24 (ATCC37051). As a method of introducing an expression vector into the host cell, any method can be used as long as a DNA is introduced into the host cell. Examples thereof can include a method using calcium ions [Proc. Natl. Acad. Sci. USA, 69, 2110 (1972)], an electroporation method, a spheroplast method, a protoplast method, a lithium acetate method, and a competent method.

As a method of expressing a nucleic acid by a host transformed with an expression vector, secretory production, fusion protein expression, or the like, can be performed according to the method described in Molecular Cloning, 2nd edition, in addition to direct expression.

The protein can be produced by, for example, culturing a host transformed with the expression vector in a culture medium, producing and accumulating the protein in the culture medium, and then collecting the protein from the culture medium. A method of culturing the host in the culture medium can be performed according to a method commonly used for culturing a host.

In the case where the host is a prokaryote such as **E.** coli or a eukaryote such as yeast, any of a natural medium and a synthetic medium may be used as a culture medium as long as it contains a carbon source, a nitrogen source, inorganic salts, and the like which can be assimilated by the host and it is a culture medium capable of efficiently culturing the host.

As the carbon source, any carbon source that can be assimilated by the transformed microorganisms may be used, and it is possible to use, for example, carbohydrate such as glucose, fructose, sucrose, or molasses, starch, or starch hydrolyzates containing the carbohydrate, organic acid such as acetic acid or propionic acid, and alcohol such as ethanol or propanol. As the nitrogen source, for example, it is possible to use an ammonium salt of inorganic or organic acid such as ammonia, ammonium chloride, ammonium sulfate, ammonium acetate, or ammonium phosphate, other nitrogen-containing compounds, peptone, meat extract, yeast extract, corn steep liquor, casein hydrolyzate, soybean cake, soybean cake hydrolyzate, and various fermentative bacteria and digested products thereof. As the inorganic salts, for example, it is possible to use monopotassium phosphate, dipotassium phosphate, magnesium phosphate, magnesium sulfate, sodium chloride, ferrous sulfate, manganese sulfate, copper sulfate, and calcium carbonate.

A prokaryote such as **E.** coli or a eukaryote such as yeast can be cultured under aerobic conditions such as shaking culture or deep aeration stirring culture. A culture temperature is, for example, 15 to 40°C. A culture time is generally 16 hours to 7 days. It is preferable to maintain a pH of the culture medium during the culture at 3.0 to 9.0. The pH of the culture medium can be adjusted using inorganic acid, organic acid, an alkali solution, urea, calcium carbonate, ammonia, or the like.

In addition, antibiotics such as ampicillin and tetracycline may be added to the culture medium during the culture, if necessary. When culturing microorganisms transformed with an expression vector using an inducible promoter as a promoter, an inducer may be added to the medium, if necessary. For example, when culturing microorganisms transformed with an expression vector using a lac promoter, isopropyl-β-D-thiogalactopyranoside or the like may be added to the medium, and when culturing microorganisms transformed with an expression vector using a trp promoter, indole acrylic acid or the like may be added to the medium.

Isolation and purification of the expressed protein can be performed by a commonly used method. For example, in the case where the protein is expressed in a dissolved state in cells, the host cells are collected by centrifugation after completion of the culture, the collected cells are suspended in an aqueous buffer, and then the host cells are disrupted using an ultrasonicator, a French press, a Manton-Gaulin homogenizer, a Dyno-Mill, or the like to obtain a cell-free extract. From the supernatant obtained by centrifuging the cell-free extract, a purified preparation can be obtained by a method commonly used for isolation and purification of a protein, that is, a solvent extraction method, a salting-out method using ammonium sulfate or the like, a desalting method, a precipitation method using an organic solvent, an anion exchange chromatography method using a resin such as diethylaminoethyl (DEAE) -Sepharose or DIAION HPA-75 (manufactured by Mitsubishi Kasei Kogyo Kabushiki Kaisha), a cation exchange chromatography method using a resin such as S-Sepharose FF (Pharmacia Corporation), a hydrophobic chromatography method using a resin such as butyl sepharose or phenyl sepharose, a gel filtration method using a molecular sieve, an affinity chromatography method, a chromatofocusing method, an electrophoresis method such as isoelectric focusing or the like, alone or in combination thereof.

In addition, in the case where the protein is expressed by formation of an insoluble matter in cells, similarly, the host cells are collected, disrupted, and then centrifuged to recover the insoluble matter of the protein as a precipitated fraction. The recovered insoluble matter of the protein can be solubilized with a protein denaturing agent. After this operation, a purified preparation of the protein can be obtained by the same isolation and purification method as described above. In the case where the protein is secreted extracellularly, the protein can be recovered from the culture supernatant. That is, a culture supernatant can be obtained by treating the culture by a method such as centrifugation, and a purified preparation can be obtained from the culture supernatant using the same isolation and purification method as described above.

(Production method of raw material composition)

In the present disclosure, the production method of the raw material composition is not particularly limited, and an example thereof may be a method including the following respective steps.

[Dissolution step]

A dissolution step is a step of dissolving a protein in a solvent (for example, a carboxylic acid such as formic acid) to obtain a protein solution.

In the dissolution step, as a protein to be dissolved (hereinafter, referred to as "hereinafter, a protein to be targeted"), a purified protein may be used, or a protein in host cells in which the protein is expressed (recombinant protein) may be used. The purified protein may be a protein purified from host cells in which the protein is expressed. In a case where the protein in the host cells is dissolved as the target protein, the host cells are brought into contact with a solvent to dissolve the protein in the host cells in the solvent. Any host cell is used as long as it is a cell in which the target protein is expressed, and may be, for example, an intact cell or a cell subjected to a treatment such as a disruption treatment. Alternatively, the cell may be a cell subjected to a simple purification treatment in advance.

A method of purifying a protein from host cells in which the protein is expressed is not particularly limited, and, for example, the methods disclosed in JP 6077570 B2 and JP 6077569 B2 can be used.

In the dissolution step, in a case where a carboxylic acid such as formic acid is used as a solvent, an esterified protein is produced by a dehydration condensation reaction between a hydroxyl group in the protein and the carboxylic acid. As the carboxylic acid, it is possible to use, for example, a monocarboxylic acid such as formic acid, acetic acid, dichloroacetic acid, trifluoroacetic acid, propionic acid, butanoic acid, isobutyric acid, pentanoic acid, caproic acid, caprylic acid, capric acid, or benzoic acid, a saturated aliphatic carboxylic acid such as capric acid, lauric acid, myristic acid, palmitic acid, stearic acid, arachidic acid, behenic acid, lignoceric acid, cerotic acid, montanic acid, melissic acid, or ceroplastic acid, an unsaturated aliphatic carboxylic acid such as undecylenic acid, oleic acid, elaidic acid, cetoleic acid, erucic acid, brassidic acid, sorbic acid, linoleic acid, linolenic acid, arachidonic acid, propiolic acid, or stearolic acid, and a dicarboxylic acid such as oxalic acid, malonic acid, succinic acid, glutaric acid, adipic acid, pimelic acid, suberic acid, azelaic acid, sebacic acid, undecanoic acid, brassylic acid, maleic acid, fumaric acid, or glutaconic acid. The carboxylic acid may be in a form of acid anhydride or acid chloride.

The dissolution step may be performed at room temperature, or may be performed to dissolve the protein in the solvent while holding the temperature at various heating temperatures. A holding time for the heating temperature is not particularly limited, but may be 10 minutes or longer, and in consideration of industrial production, 10 to 120 minutes is preferred, 10 to 60 minutes is more preferred, and 10 to 30 minutes is still more preferred. The holding time for the heating temperature may be appropriately set under a condition in which the protein is sufficiently dissolved and impurities (other than the protein to be targeted) are less dissolved.

An addition amount of the solvent added to dissolve the protein is not particularly limited as long as it is the amount in which the protein can be dissolved.

In a case where a purified protein is dissolved, the addition amount of the solvent may be 1 to 100 times, 1 to 50 times, 1 to 25 times, 1 to 10 times, or 1 to 5 times, in terms of a ratio (volume (mL)/weight (g)) of a volume (mL) of the solvent to a weight (g) of the protein (dry powder containing the protein).

In a case where a protein in host cells in which the protein is expressed is dissolved, the addition amount of the solvent may be 1 to 100 times, 1 to 50 times, 1 to 25 times, 1 to 10 times, or 1 to 5 times, in terms of a ratio (volume (mL)/weight (g)) of a volume (mL) of the solvent to a weight (g) of the host cells.

The solvent may contain an inorganic salt. Solubility of the protein can be increased by adding the inorganic salt to the solvent.

Examples of the inorganic salt that can be added to the solvent can include an alkali metal halide, an alkaline earth metal halide, an alkaline earth metal nitrate, a thiocyanate, and a perchlorate.

Examples of the alkali metal halide can include potassium bromide, sodium bromide, lithium bromide, potassium chloride, sodium chloride, lithium chloride, sodium fluoride, potassium fluoride, cesium fluoride, potassium iodide, sodium iodide, and lithium iodide.

Examples of the alkaline earth metal halide can include calcium chloride, magnesium chloride, magnesium bromide, calcium bromide, magnesium iodide, and calcium iodide.

Examples of the alkaline earth metal nitrate can include calcium nitrate, magnesium nitrate, strontium nitrate, and barium nitrate.

Examples of the thiocyanate can include sodium thiocyanate, ammonium thiocyanate, and guanidinium thiocyanate.

Examples of the perchlorate can include ammonium perchlorate, potassium perchlorate, calcium perchlorate, silver perchlorate, sodium perchlorate, and magnesium perchlorate.

These inorganic salts may be used alone or in a combination of two or more thereof.

Examples of a preferred inorganic salt can include an alkali metal halide and an alkaline earth metal halide. Specific examples of the preferred inorganic salt can include lithium chloride and calcium chloride.

An addition amount (content) of the inorganic salt may be 0.5% by mass to 10% by mass or 0.5% by mass to 5% by mass, with respect to a total mass of the solvent.

An insoluble matter may be removed from the protein solution, if necessary. That is, the production method of the raw material composition may include a step of removing an insoluble matter from the protein solution after the dissolution step, if necessary. Examples of a method of removing the insoluble matter from the protein solution can include general methods such as centrifugation and filter filtration with a drum filter, a press filter, or the like. In the case of filter filtration, the insoluble matter can be more efficiently removed from the protein solution using a filter aid such as celite or diatomaceous earth and a pre-coating agent in combination.

The protein solution contains a protein and a solvent (solvent for dissolution) that dissolves the protein. The protein solution may contain impurities included together with the protein in the dissolution step. The protein solution may be a solution for molding the raw material composition.

A content of the protein in the protein solution may be 5% by mass to 35% by mass, 5% by mass to 50% by mass, 10% by mass to 40% by mass, or 15% by mass to 35% by mass, with respect to a total amount of the protein solution.

A method of producing the protein to which the ester group is added is not particularly limited, but a production method using a carboxylic acid such as formic acid as a solvent in the above-described dissolution step may be used, or other production methods performed in the above-described dissolution step may be used. Such a method may be, for example, a production method performed in a step of reacting at least one selected from the group consisting of a carboxylic acid, acid anhydride, and acid chloride with a protein having a hydroxyl group such as serine, tyrosine, or threonine.

[Molding step]

A molding step is a step of molding a raw material composition using a protein solution. A shape of the raw material composition is not particularly limited, and examples of the raw material composition can include a fiber, a film, a molded article, a gel, a porous body, and a particle.

In the protein solution, it is preferable to adjust a concentration and viscosity of the protein depending on use of the raw material composition to be molded.

A method of adjusting the concentration of the protein in the protein solution is not particularly limited, and examples thereof can include a method of increasing the concentration of the protein by evaporating a solvent by distillation, a method using a solution having a high concentration of a protein in the dissolution step, and a method of reducing an addition amount of a solvent with respect to the amount of protein.

A viscosity suitable for spinning is generally 1,000 to 50,000 cP (centipoise, 1 cP = 1 mPa.s) at 40°C, and the viscosity can be measured using, for example, an "EMS viscometer" (trade name) manufactured by Kyoto Electronics Manufacturing Co., Ltd. When the viscosity of the protein solution is not within the above range, the viscosity of the protein solution may be adjusted to a viscosity at which spinning can be performed. The viscosity can be adjusted using the above-described method and the like. The solvent may contain an appropriate inorganic salt as exemplified above.

In a case where the raw material composition to be molded is a raw material (raw material fiber), a content (concentration) of a protein in the protein solution may be adjusted to have a concentration and viscosity at which spinning can be performed, if necessary. A method of adjusting the concentration and viscosity of the protein is not particularly limited. In addition, an example of a spinning method can include wet spinning. When the protein solution having the adjusted concentration and viscosity suitable for spinning is added to a coagulation liquid as a dope solution, the protein coagulates. In this case, since the protein solution is added to the coagulation liquid as a yarn-shaped liquid, the protein coagulates in a yarn state, and thus, a yarn (undrawn yarn) can be formed. The undrawn yarn can be formed, for example, according to a method disclosed in JP 5584932 B2.

Hereinafter, an example of the wet spinning will be described, but a spinning method is not limited, and may be dry wet spinning.

Wet spinning and drawing

(a) Wet spinning

The coagulation liquid may be any solution that can be desolventized. As the coagulation liquid, it is preferable to use a lower alcohol having 1 to 5 carbon atoms, such as methanol, ethanol, or 2-propanol, or acetone. The coagulation liquid may also contain water. A temperature of the coagulation liquid is preferably 5 to 30°C from the viewpoint of stability of spinning.

A method of adding the protein solution as a yarn-shaped liquid is not particularly limited, and an example thereof can include a method of extruding (discharging) the protein solution from a spinneret to a coagulation liquid in a desolvation bath. An undrawn yarn is obtained by coagulating the protein. An extrusion (discharging) speed in a case where the protein solution is extruded (discharged) to the coagulation liquid can be appropriately set according to a diameter of the spinneret, a viscosity of the protein solution, or the like. For example, in a case of a syringe pump having a nozzle having a diameter of 0.1 to 0.6 mm, an extrusion (discharging) speed may be 0.2 to 6.0 mL/h per hole, or may be 1.4 to 4.0 mL/h per hole, from the viewpoint of stability of spinning. A length of the desolvation bath (coagulation liquid bath) into which the coagulation liquid is introduced is not particularly limited, but may be, for example, 200 to 500 **mm. A** withdrawing speed of the undrawn yarn formed by coagulation of the protein may be, for example, 1 to 14 m/min, and a retention time may be, for example, 0.01 to 0.15 min. The withdrawing speed of the undrawn yarn may be 1 to 3 m/min from the viewpoint of efficiency of desolvation. The undrawn yarn formed by coagulation of the protein may be further drawn (pre-drawn) in a coagulation liquid, but it is preferable that the coagulation liquid is kept at a low temperature and the undrawn yarn is drawn from the coagulation liquid in a form of an undrawn yarn, from the viewpoint of suppressing vaporization of the lower alcohol used in the coagulation liquid.

(b) Drawing

A step of further drawing the undrawn yarn obtained by the above-described method can be included. The drawing may be one-stage drawing or multi-stage drawing including two or more stages. When the drawing is performed in multi stages, molecules can be aligned in multiple stages and a total draw ratio can be increased, which is suitable for producing a fiber having high toughness.

In a case where the raw material composition is a film (raw material film), the protein solution may be adjusted to have a concentration and viscosity at which the protein solution can be formed into a film, if necessary. A method of forming the raw material composition into a film is not particularly limited, and an example thereof can include a method of obtaining a film having a predetermined thickness by applying a protein solution to a flat plate having a resistance to a solvent in a predetermined thickness to form a coating film, and removing the solvent from the coating film.

As a method of forming a film having a predetermined thickness can include a casting method. In a case where a film is formed by a casting method, a raw material film (a polypeptide film) can be obtained by casting, on a flat plate, the protein solution to a thickness of several microns or more using a tool such as a doctor coat or a knife coater to form a cast film, and then removing the solvent by reduced pressure drying or immersion in a desolvation bath. The raw material film can be formed, for example, according to a method disclosed in JP 5678283 B2.

In a case where the raw material composition is a molded article (raw material molded article), a method of forming the raw material molded article is not particularly limited. For example, dried protein powder is introduced into a pressure molding machine, and pressurization and heating are performed using a hand press machine or the like, such that the dried protein power reaches a required temperature, and thus, a raw material molded article can be obtained. In addition, the raw material molded article can be formed according to a method described in the patent literature (JP 2017-539869 A, PCT/JP2016/076500).

In a case where the raw material composition is a gel (raw material gel), a method of forming the raw material gel is not particularly limited. For example, the raw material gel can be obtained by a solution production step of dissolving a dry protein in a solvent for dissolution to obtain a solution of a polypeptide and a step of substituting the solution produced in the solution generation step with a water-soluble solvent. In this case, a step of pouring the solution into a mold to mold the raw material gel into a predetermined shape is performed between the solution production step and the step of substituting the solvent for dissolution with the water-soluble solvent, or cutting of the raw material gel into a predetermined shape can be performed after the step of substituting the solvent for dissolution with the water-soluble solvent. In addition, the raw material gel can be formed, for example, according to a method disclosed in JP 05782580 B2.

In a case where the raw material composition is a porous body (raw material porous body), the protein solution may be adjusted to have the concentration and viscosity at which the protein solution can be formed into a porous body. A method of forming the raw material porous body is not particularly limited. Examples thereof can include a method of obtaining a raw material porous body by adding an appropriate amount of a foaming agent to the protein solution adjusted to have a concentration and viscosity suitable for forming the protein solution into a porous body and removing the solvent, and a method described in JP 5796147 B2.

In a case where the raw material composition is a particle (raw material particle), a method of forming the particle is not particularly limited. The raw material particle can be obtained by, for example, a method including a step of obtaining an aqueous solution of a protein by substituting a solvent in a dope solution with a water-soluble solvent using the above-described dope solution, and a step of drying the aqueous solution of the protein. The water-soluble solvent is a solvent containing water, and examples thereof can include water, a water-soluble buffer, and a physiological salt solution. The step of substituting the solvent with the water-soluble solvent is preferably performed by a method in which a dope solution is placed in a dialysis membrane, the dialysis membrane is immersed in a water-soluble solvent, and the water-soluble solvent is replaced at least once. Specifically, it is more preferable that the dope solution is placed in the dialysis membrane, the dialysis membrane is left to stand in the water-soluble solvent in an amount of 100 times or more the amount of the dope solution (dose) for 3 hours, and the replacement of the water-soluble solvent is performed three times or more in total. Any dialysis membrane may be used as long as it does not allow the protein to permeate, and for example, the dialysis membrane may be a cellulose dialysis membrane or the like. The amount of the solvent in the dope solution can be adjusted to approach zero by repeating the substitution with the water-soluble solvent. The dialysis membrane may not be used in the latter half of the step of substituting the solvent with the water-soluble solvent. In the step of drying the aqueous solution of the protein, vacuum freeze-drying is preferable used. A degree of vacuum during the vacuum freeze-drying is preferably 200 pascal (Pa) or less, more preferably 150 Pa or less, and still more preferably 100 Pa or less. A moisture content in the freeze-dried particles is preferably 5.0% or less and more preferably 3.0% or less.

In a case where the raw material composition is a fiber (raw material fiber), the raw material fiber may be a hank state or a cloth state.

A lower limit of a fiber diameter of the raw material fiber may be, for example, 10 $\mu$m or more, 15 $\mu$m or more, 20 $\mu$m or more, more than 25 $\mu$m, 28 $\mu$m or more, 30 $\mu$m or more, 32 $\mu$m or more, 34 $\mu$m or more, 35 $\mu$m or more, 36 $\mu$m or more, 38 $\mu$m or more, or 40 $\mu$m or more. An upper limit of the fiber diameter of the raw material fiber is preferably 120 $\mu$m or less.

The fiber diameter of the raw material fiber may be 10 $\mu$m to 120 $\mu$m, 10 $\mu$m to 40 $\mu$m, 10 $\mu$m to 30 $\mu$m, 10 $\mu$m to 20 $\mu$m, 10 $\mu$m to 25 $\mu$m, 15 $\mu$m to 25 $\mu$m, more than 25 $\mu$m to 120 $\mu$m, 30 $\mu$m to 120 $\mu$m, 35 $\mu$m to 120 $\mu$m, 40 $\mu$m to 120 $\mu$m, 45 $\mu$m to 120 $\mu$m, 48 $\mu$m to 120 $\mu$m, 50 $\mu$m to 120 $\mu$m, 55 $\mu$m to 120 $\mu$m, 60 $\mu$m to 120 $\mu$m, 65 $\mu$m to 120 $\mu$m, 55 $\mu$m to 100 $\mu$m, 55 $\mu$m to 80 $\mu$m, or 60 $\mu$m to 80 um. When the fiber diameter is 10 $\mu$m or more, it is possible to reduce the shrinkage by contact with water. When the fiber diameter is 120 $\mu$m or less, desolvation when forming the fiber can be further efficiently performed, and a hydrolysis reaction rate of the ester group can be further increased.

(Production method of protein composition)

A production method according to the present invention includes a step of hydrolyzing an ester group by bringing a raw material composition containing an esterified protein (a protein having an ester group) into contact with an acidic or basic medium (hereinafter, also referred to as a "hydrolysis step"). By performing the hydrolysis step for the ester group, it is possible to perform a shrinkage treatment on the raw material composition at the same time, thereby obtaining a shrink-proof effect.

[Hydrolysis step]

The medium is a medium containing water. The medium containing water is an aqueous solution or water vapor. The medium containing water is an acidic or basic medium. The acidic medium has acidity. For example, the acidic medium is an acidic aqueous solution or acidic water vapor having a pH of lower than 7, and the acidic medium is preferably an acidic aqueous solution or acidic water vapor having a pH of 1 or higher, from the viewpoint of controlling hydrolysis and side reaction of a molecule chain. The basic medium has alkalinity. For example, the basic medium is a basic aqueous solution or basic water vapor having a pH of higher than 7, and the basic medium is preferably a basic aqueous solution or basic water vapor having a pH of 12 or lower, from the viewpoint of controlling hydrolysis and side reaction of a molecule chain.

The temperature of the medium containing water is 40°C to 180°C. The temperature of the medium containing water may be, for example, 50°C or higher, 60°C or higher, 70°C or higher, 80°C or higher, 85°C or higher, 90°C or higher, or 95°C or higher. The temperature of the medium containing water is preferably a boiling point or lower.

The method includes bringing a raw material composition (for example, a raw material fiber) into contact with an acidic or basic aqueous solution. In this case, the amount of acidic or basic substances is preferably 0.1% by mass or more, and more preferably 1.0% by mass or more, with respect to the total amount of the protein solution.

The acidic substances that can be used in hydrolysis may be, but not particularly limited to, an inorganic acid or organic acid. Examples of the inorganic acid can include hydrochloric acid, sulfuric acid, nitric acid, phosphoric acid, and boric acid. Examples of the organic acid can include carboxylic acid and sulfonic acid. Examples of the carboxylic acid can include a monocarboxylic acid such as formic acid, acetic acid, dichloroacetic acid, trifluoroacetic acid, propionic acid, butanoic acid, isobutyric acid, pentanoic acid, caproic acid, caprylic acid, capric acid, or benzoic acid, a saturated aliphatic carboxylic acid such as capric acid, lauric acid, myristic acid, palmitic acid, stearic acid, arachidic acid, behenic acid, lignoceric acid, cerotic acid, montanic acid, melissic acid, or ceroplastic acid, an unsaturated aliphatic carboxylic acid such as undecylenic acid, oleic acid, elaidic acid, cetoleic acid, erucic acid, brassidic acid, sorbic acid, linoleic acid, linolenic acid, arachidonic acid, propiolic acid, or stearolic acid, and a dicarboxylic acid such as oxalic acid, malonic acid, succinic acid, glutaric acid, adipic acid, pimelic acid, suberic acid, azelaic acid, sebacic acid, undecanoic acid, brassylic acid, maleic acid, fumaric acid, or glutaconic acid. The carboxylic acid may be in a form of acid anhydride or acid chloride. Examples of the sulfonic acid can include methanesulfonic acid, trifluoromethanesulfonic acid, benzenesulfonic acid, and p-toluenesulfonic acid.

The basic substances that can be used in hydrolysis may be, but not particularly limited to, an inorganic base or organic base. The inorganic base is not particularly limited as long as it is soluble in water. Examples of the inorganic base can include potassium hydroxide, sodium hydroxide, sodium bicarbonate, and sodium carbonate. Examples of the organic base can include alkylamine such as ammonia, methylamine, dimethylamine, trimethylamine, ethylamine, diethylamine, and triethylamine, and primary to tertiary amines such as aminoethanol, methylaminoethanol, dimethylaminoethanol, ethylaminoethanol, diethylaminoethanol, and diethanolamine.

The hydrolysis of the ester group is an equilibrium reaction. An acid similar to the acid produced when the ester group is dissociated can be used as an acid required for hydrolysis, but it is preferable not to use such an acid.

The hydrolysis proceeds under an acidic condition or basic condition. An acidic condition in which a pH is 1 to 6 or a basic condition in which a pH is 8 to 14 is preferred, and the pH can be adjusted by acidic substances or basic substances to be used.

In the present disclosure, in a case where a basic aqueous solution is used, the pH is preferably higher than 8, and preferably 12 or lower, from the viewpoint of the above-described reasons.

In a case where the basic aqueous solution is used, after the carboxylic acid is dissociated by hydrolysis, a carboxylic acid anion is formed. In this case, the electrophilicity is lost, and thus, a reverse reaction is less likely to occur. The pH of the basic aqueous solution is preferably 8 to 14, from the viewpoint of implementing high reactivity without heating, and the pH of the basic aqueous solution is more preferably higher than 8, from the viewpoint of the hydrolysis reaction rate.

Since the hydrolysis of the ester group proceeds rapidly in the acidic or basic aqueous solution, a reaction time is not limited, but is preferably longer than 1 minute, from the viewpoint of sufficiently removing the ester group.

The temperature at which the hydrolysis is performed is 40°C or higher, 50°C or higher, 60°C or higher, 70°C or higher, 80°C or higher, 85°C or higher, 90°C or higher, or 95°C or higher. The temperature at which the hydrolysis is performed is 180°C or lower, and is preferably a boiling point or lower.

In the present disclosure, a contact time with the medium containing water may be, for example, 5 minutes or longer, 10 minutes or longer, 20 minutes or longer, or 30 minutes or longer. The contact time with the medium containing water may be 90 minutes or shorter, 60 minutes or shorter, or 40 minutes or shorter.

In the present disclosure, the acidic substances or basic substances may remain in the protein composition (for example, the protein fiber) taken out from the aqueous solution used for the hydrolysis, and the acidic substances or basic substances may cause breakage of the molecular chains. In order to prevent the breakage of the molecular chains, the production method of the protein composition (for example, the protein fiber) may further include a step of removing the remaining acid substances or basic substances. Examples of the step of removing the remaining acid substances or basic substances can include a step of washing the protein composition (for example, the protein fiber) with water and a step of neutralizing the protein composition (for example, the protein fiber).

In the present disclosure, in a case where the protein composition is a protein fiber, the protein fiber obtained in the hydrolysis step may have a shrinkage rate of -5% to +5%, the shrinkage rate being defined by the following Equation (1). Details of the shrinkage rate will be described below, the shrinkage rate defined by the following Equation (1):

Shrinkage rate = {1 - (length of protein fiber when dried from wet state/length of protein fiber before in wet state)} × 100 [%].

[Shrinking step]

The production method according to the present disclosure may further include a step of irreversibly shrinking the raw material composition (for example, the raw material fiber) before and/or after the hydrolysis step (hereinafter, also referred to as a "shrinking step"). By performing the hydrolysis step, the raw material composition (for example, the raw material fiber) can be simultaneously subjected to a shrinkage (shrink-proof) treatment, and thus, one or both of the shrinking steps can be omitted. In the shrinking step, the raw material composition may be irreversibly shrunk by bringing the raw material composition (for example, the raw material fiber) into contact with water, or the raw material composition may be irreversibly shrunk by heating and relaxing the raw material composition (for example, the raw material fiber). In the case where the raw material composition (for example, the raw material fiber) is irreversibly shrunk by bringing the raw material fiber into contact with water, the irreversibly shrunk composition may be dried and further shrunk.

(Shrinking step by contact with water (contact step))

The raw material fiber (the fiber containing a protein) according to the present disclosure has the property of shrinking when being in contact (being wetted) with water below the boiling point. Therefore, in the shrinking step, the raw material fiber is brought into contact with water, such that a protein fiber having a shrinkage history of being irreversibly shrunk can be obtained. A step of irreversibly shrinking the raw material fiber by bringing the raw material fiber into contact with water is hereinafter referred to as a "contact step".

It is considered that the irreversible shrinkage of the raw material fiber (the fiber containing the protein) in the contact step occurs, for example, for the following reasons. That is, one reason is considered to be due to a primary structure of the raw material fiber (the fiber containing the protein). Another reason is considered to be that, for example, in the raw material fiber (the fiber containing the protein) having a residual stress due to drawing or the like in the production process, the residual stress is relieved by water entering between fibers or into the fiber.

In the contact step, the raw material fiber before being brought into contact with water after spinning is brought into contact with water to bring the raw material fiber into a wet state. The wet state refers to a state in which at least a part of the raw material fiber is wetted with water. Therefore, the raw material fiber can be shrunk regardless of an external force. This shrinkage is irreversible.

A temperature of the water coming into contact with the raw material fiber in the contact step may be lower than a boiling point. Therefore, handleability, workability in the shrinking step, and the like are improved. In addition, a lower limit of the temperature of the water is preferably 10°C or higher, more preferably 40°C or higher, still more preferably 70°C or higher, still more preferably 80°C or higher, and particularly preferably 90°C or higher, from the viewpoint of sufficiently shortening the shrinkage time. An upper limit of the temperature of the water is preferably the boiling point or lower.

In the contact step, a method of bringing the raw material fiber into contact with water is not particularly limited. Examples of the method can include a method of immersing the raw material fiber in water, a method of spraying water onto the raw material fiber at room temperature or in a heated steam state, and a method of exposing the raw material fiber to a high humidity environment filled with water vapor. Among these methods, the method of immersing the raw material fiber in water is preferred in the contact step, since the shrinkage time can be effectively shortened and the processing equipment can be simplified.

In the contact step, when the raw material fiber is brought into contact with water in a relaxed state, the raw material fiber may be not only shrunk but also be curled to be wavy. In order to prevent the occurrence of curling, for example, the contact step may be performed in a state where the raw material fiber is not relaxed, for example, in a state where the raw material fiber is brought into contact with water while being tensioned in an axial direction of the fiber to the extent that a tension is not applied.

(Drying step)

The production method according to the present disclosure may further include a drying step. The drying step is a step of drying the raw material fiber subjected to the contact step (or the protein fiber obtained through the contact step). Drying may be, for example, natural drying, or forced drying using drying equipment. As the drying equipment, any known drying equipment of contact type or non-contact type can be used. In addition, a drying temperature is not limited as long as it is lower than a temperature at which the protein contained in the raw material fiber is degraded or the raw material fiber is thermally damaged. In general, the drying temperature is a temperature in a range of 20 to 150°C, and is preferably a temperature in a range of 50 to 100°C. When the temperature is in this range, the fiber is more quickly and efficiently dried without thermal damage to the fiber or degradation of the protein contained in the fiber. A drying time is appropriately set depending on the drying temperature or the like, and for example, a time during which the influence on the quality and physical properties of the protein fiber due to overdrying can be eliminated as much as possible is employed.

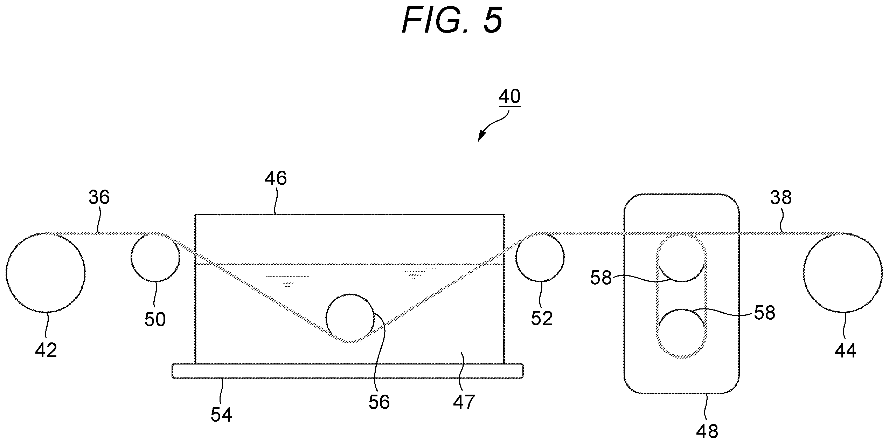

Fig. 5 is an explanation diagram schematically illustrating an example of a production apparatus for producing a protein fiber. A production apparatus 40 illustrated in Fig. 5 includes a feed roller 42 for feeding the raw material fiber, a winder 44 for winding a protein fiber 38, a water bath 46 for performing the contact step, and a dryer 48 for performing the drying step.

More specifically, the feed roller 42 can be loaded with a wound product of a raw material fiber 36, and the raw material fiber 36 can be continuously and automatically fed from the wound product of the raw material fiber 36 by rotation of an electric motor or the like (not illustrated). The winder 44 can continuously and automatically wind the protein fiber 38 produced through the contact step and the drying step after being fed out from the feed roller 42 by the rotation of the electric motor (not illustrated). Here, a feed speed of the raw material fiber 36 by the feed roller 42 and a winding speed of the protein fiber 38 by the winder 44 can be controlled independently of each other.

The water bath 46 and the dryer 48 are arranged between the feed roller 42 and the winder 44 on the upstream side and the downstream side in a feed direction of the raw material fiber 36, respectively. The production apparatus 40 illustrated in Fig. 5 includes relay rollers 50 and 52 relaying the raw material fiber 36 before and after the contact step which moves from the feed roller 42 toward the winder 44.

The water bath 46 includes a heater 54, and water 47 heated by the heater 54 is accommodated in the water bath 46. In addition, in the water bath 46, a tension roller 56 is installed in a state of being immersed in the water 47. Accordingly, the raw material fiber 36 fed from the feed roller 42 moves toward the winder 44 while being immersed in the water 47 in a state of being wound around the tension roller 56 in the water bath 46. An immersion time of the raw material fiber 36 in the water 47 is appropriately controlled according to a moving speed of the raw material fiber 36.

The dryer 48 has a pair of hot rollers 58. The pair of hot rollers 58 can be wound with the raw material fiber 36 which is released from the water bath 46 and moves toward the winder 44. Accordingly, the raw material fiber 36 immersed in the water 47 in the water bath 46 is heated by the pair of hot rollers 58 in the dryer 48, dried, and then further fed toward the winder 44.

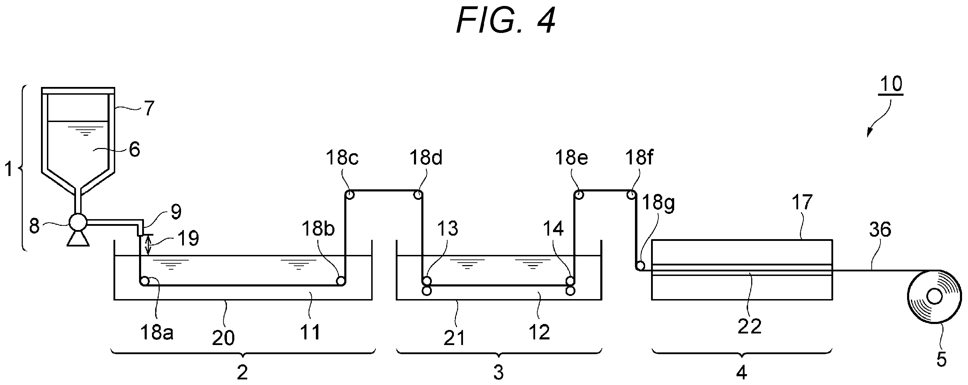

When the protein fiber 38 is produced using the production apparatus 40 having such a structure, first, for example, the wound product of the raw material fiber 36 spun using the spinning apparatus 10 illustrated in Fig. 4 is mounted on the feed roller 42. Next, the raw material fiber 36 is continuously fed from the feed roller 42 and immersed in the water 47 in the water bath 46. In this case, for example, the winding speed of the winder 44 is slower than the feed speed of the feed roller 42. Accordingly, since the raw material fiber 36 is shrunk due to contact with the water 47 in a state of not being relaxed between the feed roller 42 and the winder 44, the occurrence of curling can be prevented. The raw material fiber 36 is irreversibly shrunk due to contact with the water 47.

Next, the raw material fiber 36 after being brought into contact with the water 47 (or the protein fiber 38 produced through contact with the water 47) is heated by the pair of hot rollers 58 of the dryer 48. Accordingly, the raw material fiber 36 after being brought into contact with the water 47 (or the protein fiber 38 produced through contact with the water 47) can be dried and further shrunk. In this case, a ratio of the feed speed of the feed roller 42 and the winding speed of the winder 44 can be controlled so that the length of the protein fiber 38 is not changed. Then, the obtained protein fiber 38 is wound around the winder 44 to obtain the wound product of the protein fiber 38.

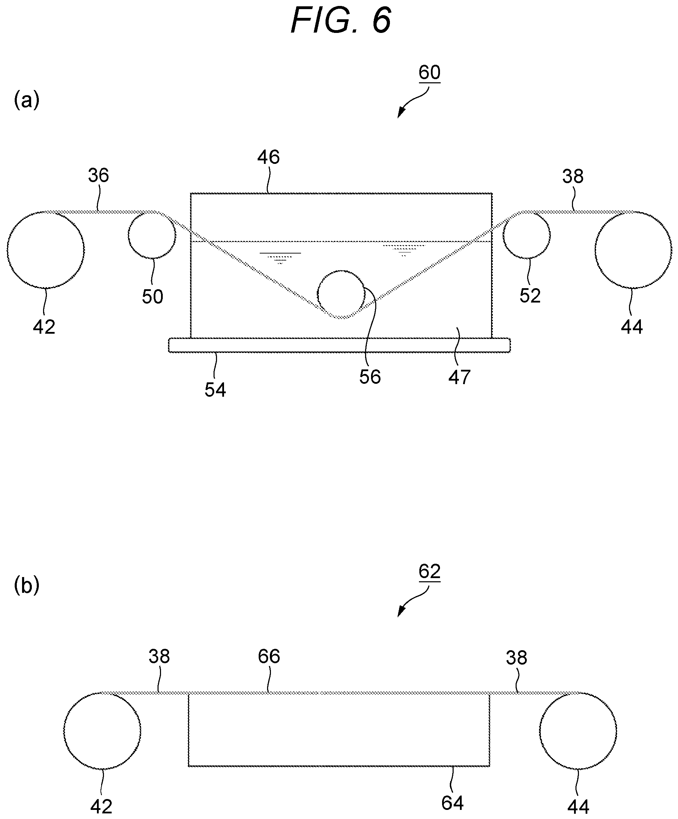

Instead of the pair of hot rollers 58, the raw material fiber 36 obtained after being brought into contact with the water 47 may be dried using drying equipment having only a heat source, such as a dry heat plate 64 as illustrated in Fig. 6(b). Also, in this case, by adjusting a relative speed between the feed speed of the feed roller 42 and the winding speed of the winder 44 in the same manner as in the case of using the pair of hot rollers 58 as the drying equipment, the length of the protein fiber cannot be changed. Here, the drying means includes the dry heat plate 64. In addition, the dryer 48 is optional.

As described above, the protein fiber 38 to be targeted can be automatically, continuously, and extremely easily produced using the production apparatus 40.

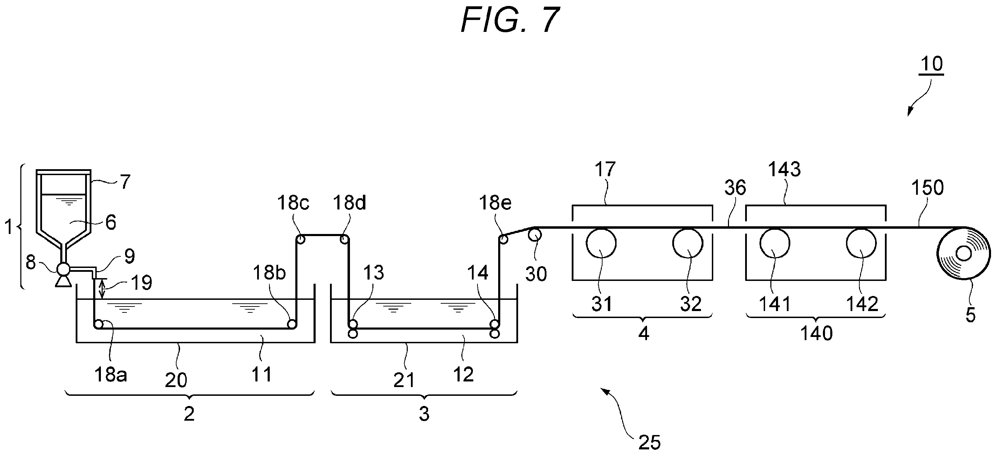

Fig. 6 is an explanation diagram schematically illustrating another example of a production apparatus for producing a protein fiber. Fig. 6(a) illustrates a processing device that is included in the production apparatus and that performs the contact step. Fig. 6(b) illustrates a drying device that is included in the production apparatus and that performs the drying step. The production apparatus illustrated in Fig. 7 includes a processing device 60 for performing the contact step on the raw material fiber 36, and a drying device 62 for drying the raw material fiber 36 after the contact step (or the protein fiber 38 produced through the contact step), and the production apparatus has a structure in which these devices are installed independently of each other.

More specifically, the processing device 60 illustrated in Fig. 6(a) has a structure in which the feed roller 42, the water bath 46, and the winder 44 are arranged in order from the upstream side to the downstream side in a moving direction of the raw material fiber 36. Such a processing device 60 is designed to allow the raw material fiber 36 fed from the feed roller 42 to be immersed in the water 47 in the water bath 46 and to be shrunk. In addition, the obtained protein fiber 38 is wound around the winder 44. In this case, for example, the winding speed of the winder 44 is slower than the feed speed of the feed roller 42. Accordingly, since the raw material fiber 36 is shrunk due to contact with the water 47 in a state of being relaxed between the feed roller 42 and the winder 44, it is possible to prevent the fiber from being tensioned. The raw material fiber 36 is irreversibly shrunk due to contact with the water 47.

The drying device 62 illustrated in Fig. 6(b) includes a feed roller 42, a winder 44, and a dry heat plate 64. The dry heat plate 64 is arranged between the feed roller 42 and the winder 44 so that a dry heat surface 66 comes into contact with the protein fiber 38 and extends along in the moving direction thereof. In the drying device 62, as described above, the length of the protein fiber 38 cannot be changed by, for example, controlling a ratio of a feed speed of the feed roller 42 and a winding speed of the winder 44.

By using the production apparatus having such a structure, the protein fiber 38 is obtained by shrinking the raw material fiber 36 by the processing device 60, and then, the protein fiber 38 can be dried by the drying device 62.

The feed roller 42 and the winder 44 may be omitted from the processing device 60 illustrated in Fig. 6(a), and the processing device may include only the water bath 46. In a case where the production apparatus including such a processing device is used, for example, the protein fiber is produced in a so-called batch system. In addition, the drying device 62 illustrated in Fig. 6(b) is optional.

(Shrinking step by heating and relaxation)

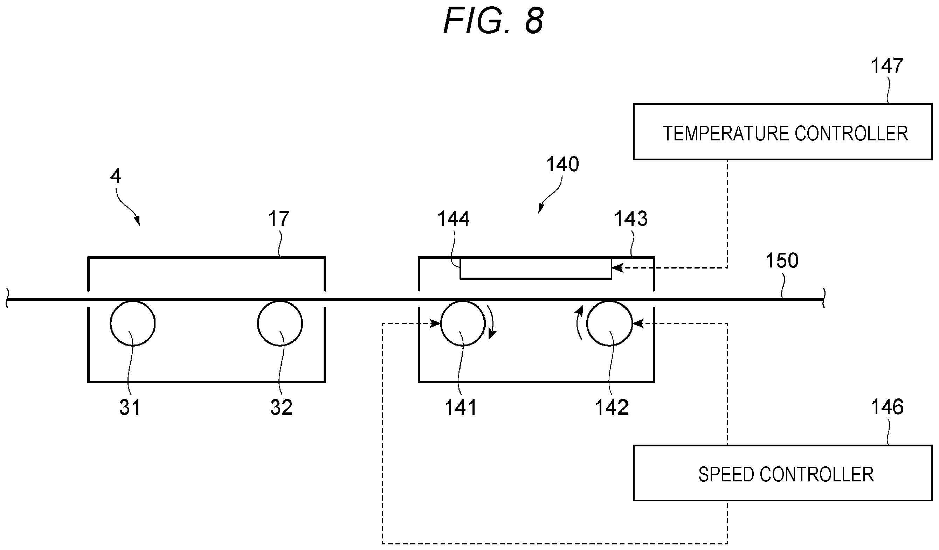

The shrinking step of irreversibly shrinking the raw material fiber may be performed by heating and relaxing the raw material fiber. The heating and relaxation of the raw material fiber can be performed by heating the raw material fiber and relaxing and shrinking the heated raw material fiber. Hereinafter, in the shrinkage performed by the heating and relaxation of the raw material fiber, the step of heating the raw material fiber is referred to as a "heating step", and the step of relaxing and shrinking the heated raw material fiber is referred to as a "relaxation and shrinking step". The heating step and the relaxation and shrinking step can be performed by, for example, a high temperature heating relaxation device 140 illustrated in Fig. 7 or Fig. 8.

(Heating step)

In the heating step, the heating temperature of the raw material fiber 36 is preferably equal to or higher than a softening temperature of the protein used in the raw material fiber 36. In the specification, the softening temperature of the protein is a temperature at which shrinkage is initiated due to stress relaxation of the raw material fiber 36. In the heating and relaxation shrinking at the temperature equal to or higher than the softening temperature of the protein, the fiber is shrunk to the extent that it cannot be obtained simply by removing moisture in the fiber. As a result, a residual stress in the fiber generated by drawing in the spinning process can be removed.

An example of a temperature corresponding to the softening temperature can include 180°C. In a case where the heating and relaxation shrinking is performed in a high temperature range of 180°C or higher, as a relaxation ratio becomes large or the temperature becomes high, the residual stress in the raw material fiber can be more efficiently removed. Accordingly, the heating temperature of the raw material fiber 36 is preferably 180°C or higher, more preferably 180°C to 280°C, still more preferably 200°C to 240°C, and particularly preferably 220°C to 240°C.

A heating time in the heating step, that is, a retention time in a high temperature heating furnace 143 is preferably 60 seconds or shorter, more preferably 30 seconds or shorter, and still more preferably 5 seconds or shorter, from the viewpoint that stretching of the fiber obtained after the heat treatment is not impaired. It is considered that the length of the heating time does not significantly affect the stress. When the heating time at the heating temperature of 200°C is 5 seconds or shorter, a deterioration of stretching of the fiber obtained by the heat treatment can be prevented.

(Relaxation and shrinking step)

In the relaxation and shrinking step, the relaxation ratio preferably exceeds 1 time, more preferably 1.4 times or more, still more preferably 1.7 times or more, and particularly preferably 2 times or more. The relaxation ratio is a ratio of the feed speed to the winding speed of the raw material fiber 36, and more specifically, a ratio of a feed speed by a feed roller 141 to a winding speed by a winding roller 142.

In the heating and relaxation method performed using the high temperature heating relaxation device 140, the heating step and the relaxation and shrinking step may be separately performed as long as the raw material fiber 36 can be relaxed in a heated state. That is, the heating device may be a device separated from and independent of a relaxation device. In this case, the relaxation device is provided at a subsequence stage of the heating device (the downstream side in the moving direction of the raw material fiber 36) so that the relaxation and shrinking step is performed after the heating step.

The heating and relaxation step may be performed on the raw material fiber separately from the production process of the raw material fiber. That is, the same device as the high temperature heating relaxation device 140 may be provided as an independent device separated from a spinning apparatus 25. A method in which the separately produced raw material fiber 36 is set to the feed roller and the raw material fiber is fed from the feed roller may be adopted. The heating and relaxation step may be performed on one raw material fiber or a plurality of bundled fibers.

(Crosslinking step)

A crosslinking step of performing chemical crosslinking between polypeptide molecules in the protein fiber having a shrinkage history of being irreversibly shrunk, which is obtained as described above, or in the raw material fiber before being irreversibly shrunk may be further performed. Examples of a functional group which can be crosslinked can include an amino group, a carboxyl group, a thiol group, and a hydroxyl group. For example, an amino group of a lysine side chain contained in a polypeptide can be crosslinked through an amide bond by dehydration condensation with a carboxyl group of a glutamic acid or asparaginic acid side chain. The crosslinking may be performed by a dehydration condensation reaction under vacuum heating or may be performed using a dehydration condensation agent such as carbodiimide.

The crosslinking between polypeptide molecules may be performed using a crosslinking agent such as carbodiimide or glutaraldehyde or may be performed using an enzyme such as transglutaminase. The carbodiimide is a compound represented by General Formula: $R_1N=C=NR_2$ (where $R_1$ and $R_2$ each independently represent an organic group having an alkyl group or cycloalkyl group having 1 to 6 carbon atoms). Specific examples of the carbodiimide can include 1-ethyl-3-(3-dimethylaminopropyl)carbodiimide hydrochloride (EDC), N,N'-dicyclohexylcarbodiimide (DCC), 1-cyclohexyl-3-(2-morpholinoethyl)carbodiimide, and diisopropyl carbodiimide (DIC). Among them, EDC and DIC are preferred since they have a high ability to form an amide bond between polypeptide molecules and facilitate a crosslinking reaction.

A crosslinking treatment is preferably performed by applying a crosslinking agent to the fiber and performing crosslinking by vacuum heating drying. As the crosslinking agent, a pure product may be applied to the fiber. Alternatively, the crosslinking agent may be applied to the fiber by diluting a pure product with a lower alcohol having 1 to 5 carbon atoms and a buffer or the like to a concentration of 0.005 to 10% by mass. The crosslinking treatment is preferably performed at a temperature of 20 to 45°C for 3 to 42 hours. By the crosslinking treatment, a higher stress (strength) can be imparted to the fiber.

In the present disclosure, the raw material composition is a fiber, the medium is an aqueous solution, and the production method of the protein composition may further include a crimping step of crimping the fiber by bringing the fiber into contact with the aqueous solution. When the production method includes the crimping step, the removal of the ester group and the shrinkage of the fiber can be simultaneously performed by contact of the fiber with water.

In the present disclosure, the raw material composition is a fiber, the medium is an aqueous solution, and the production method of the protein composition may further include a shrink-proof step of shrink-proofing the fiber by bringing the fiber into contact with the aqueous solution. In the case of the fiber shrunk by contact with water first, a degree of shrinkage when being in contact with water is suppressed than a fiber which is not brought into contact with water. That is, when the production method includes the shrink-proof step, the removal of the ester group and the shrink-proof of the fiber can be simultaneously performed by contact of the fiber with water.

A production method of a protein composition according to another disclosure includes a step of hydrolyzing an ester group by bringing a raw material composition containing an esterified protein and an acid or a base into contact with water vapor.

[Protein composition]

A protein composition according to the present disclosure contains a protein and has a hydrolysis history of an ester group. The protein is a structural protein. The protein composition may be a fiber (protein fiber), a film (protein film), a molded article (protein molded article), a gel (protein gel), a porous body (protein porous body), and a particle (protein particle).

The hydrolysis history is preferably a history in which the ester group is hydrolyzed by bringing the protein composition into contact with an acidic or basic medium containing water.

The temperature of the medium containing water is 40°C or higher, 50°C or higher, 60°C or higher, 70°C or higher, 80°C or higher, 85°C or higher, 90°C or higher, or 95°C or higher. The temperature of the medium containing water is 180°C or lower, and is preferably a boiling point or lower.

In the present disclosure, the ester group may be included in formic acid ester, acetic acid ester, propionic acid ester, or the like, and the ester group is preferably included in formic acid ester.

The protein composition may further have a shrinkage history of being irreversibly shrunk. The "irreversible shrinkage" of the protein composition (for example, the protein fiber) refers to shrinkage when first being brought into contact with water after spinning, and corresponds to shrinkage (shrink-proof) by contact with water during the hydrolysis treatment of the ester group.

<Shrinkage rate>

In a case where the protein composition is a protein fiber, a shrinkage rate of the protein fiber is preferably -5% to +5%, the shrinkage rate being defined by the following Equation (1):

Shrinkage rate [%] = {1 - (length of protein fiber when dried from wet state/length of protein fiber before in wet state)} × 100.

Shrinkability by contact of the fiber with water can be evaluated using, for example, the shrinkage rate determined by Equation (1) as an index. "Length of protein fiber before in wet state" and "length of protein fiber when dried from wet state" can be measured by, for example, the following method.

A plurality of protein fibers having a length of about 30 cm are bundled to obtain a fiber bundle having a fineness of 150 denier. The length can be used as "length of protein fiber before in wet state". The fiber bundle is immersed (wetted) in water at 40°C for 15 minutes, and the immersed fiber bundle is dried at room temperature for 2 hours. After drying, a length of the fiber bundle is measured. The length when dried can be used as "length of protein fiber when dried from wet state".

In the protein fiber, it is preferable that such shrinkage is small, and it is particularly preferable that shrinkage of a product such as a fabric formed of a protein fiber is small.

The shrinkage rate defined by Equation (1) may be, for example, -4.5% to +4.5%, -4% to +4%, -3.5% to +3.5%, -3% to +3%, -2% to +2%, -1% to +1%, 0% to +5%, 0% to +4%, 0% to +3%, 0% to +2%, or 0% to +1%.

The protein fiber may have various sectional shapes depending on the shape of the spinneret, but the sectional shape of the protein fiber may be a circular shape or an elliptical shape.

The protein fiber may have a matte-toned appearance or a glossy appearance. A desolvation speed and/or coagulation speed in the spinning process are appropriately adjusted, such that the glossy of the appearance of the fiber can be adjusted. In the present specification, the "matte-toned appearance" means that an appearance is low-gloss.

In addition, a lower limit of a fiber diameter of the protein fiber is not particularly limited, but may be 10 $\mu$m or more, 15 $\mu$m or more, 20 $\mu$m or more, 25 $\mu$m or more, 28 $\mu$m or more, 30 $\mu$m or more, 32 $\mu$m or more, 33 $\mu$m or more, more than 33

μm, 34 μm or more, 35 μm or more, 36 μm or more, 38 μm or more, or 40 μm or more. When the fiber diameter is 10 μm or more, it is possible to further increase productivity.

An upper limit of the fiber diameter of the protein fiber is not particularly limited, but may be 120 μm or less. The fiber diameter of the protein fiber may be 10 μm to 120 μm, 12 μm to 40 μm, 10 μm to 40 μm, 12 μm to 30 μm, 10 μm to 30 μm, 10 μm to 20 μm, 12 μm to 20 μm, 20 μm to 30 μm, more than 25 μm to 120 μm, 30 μm to 120 μm, more than 33 μm to 120 μm, 34 μm to 120 μm, 35 μm to 120 μm, 40 μm to 120 μm, 45 μm to 120 μm, 48 μm to 120 μm, 50 μm to 120 μm, 55 μm to 120 μm, 60 μm to 120 μm, 65 μm to 120 μm, 55 μm to 100 μm, 55 μm to 80 μm, or 60 μm to 80 μm. When the fiber diameter is 10 μm or more, it is possible to further increase productivity. When the fiber diameter is 120 μm or less, it is possible to further increase the hydrolysis reaction rate of the ester group.

It is preferable that the protein fiber according to the present disclosure has a small change in fiber diameter before and after the shrinking step of irreversibly shrinking the raw material fiber. Specifically, it is preferable that the protein fiber has a fiber diameter of less than ±20% of the fiber diameter of the raw material fiber before being irreversibly shrunk. The fiber diameter of the protein fiber with respect to the fiber diameter of the raw material fiber is preferably less than ±20%, and may be ±19% or less, ±18% or less, ±17% or less, ±16% or less, ±15% or less, less than ±15%, ±12% or less, ±10% or less, less than ±10%, ±5% or less, less than ±5%, ±4% or less, less than ±4%, ±3% or less, less than ±3%, ±2% or less, less than ±2%, ±1% or less, less than ±1%, ±0.9% or less, ±0.8% or less, ±0.7% or less, ±0.7% or less, ±0.6% or less, ±0.5% or less, less than ±0.5%, or ±0.45% or less. The value can be determined by a calculation formula of (fiber diameter of protein fiber - fiber diameter of raw material fiber)/fiber diameter of raw material fiber × 100%.

[Ester group removal method]

An ester group removal method according to the present disclosure includes a step of bringing a raw material composition containing an esterified protein (a protein having an ester group) into contact with an acidic or basic medium containing water.

In the present disclosure, the same aspects as those described in the above-described production method of the protein composition can be applied to the protein and the raw material composition.

In the present disclosure, the protein composition (for example, the protein fiber) obtained in the above step may have a shrinkage rate of -5% to +5%, the shrinkage rate being defined by the following Equation (1). The same aspects as those described in the above-described protein composition can be applied to the shrinkage rate defined by the following Equation (1):

Shrinkage rate = {1 - (length of protein fiber when dried from wet state/length of protein fiber before in wet state)} × 100 [%].

In the present disclosure, the same aspects as those described in the above-described production method of the protein composition can be applied to the temperature and properties of the medium containing water and the contact time with the medium containing water.

[Product]

The protein composition (for example, the protein fiber) according to the present disclosure can be applied to various products. Examples of the product can include a fiber, a yarn, a fabric, a knitted fabric, a braided fabric, a non-woven fabric, a paper, cotton, and a clothing product. Examples of the fiber can include a long fiber, a short fiber, a monofilament, and a multifilament. Examples of the yarn can include a spun yarn, a twisted yarn, a false twisted yarn, a processed yarn, a blended yarn, and a blended spun yarn. Furthermore, it is possible to produce, from these fibers or yarns, a fabric such as a woven fabric, a knitted fabric, a braided fabric, or a non-woven fabric, a paper, cotton, and the like. These products can be produced by a known method. In addition, the protein composition according to the present disclosure, which can also be applied to high strength applications such as a rope, a surgical suture, a flexible stop for electrical components, and further, a physiologically active material for implantation (for example, artificial ligament and aortic band) can be applied to a yarn (a spun yarn, a twisted yarn, a false twisted yarn, a processed yarn, a blended yarn, a blended spun yarn, or the like), a woven fabric (fabric), a knitted fabric, a braided fabric, a non-woven fabric, a paper, cotton, and the like. In addition, the modified fibroin fiber can also be applied to high strength applications such as a rope, a surgical suture, a flexible stop for electrical components, and a physiologically active material for implantation (for example, artificial ligament and aortic band). These fibers can be produced based on a known method.

Examples

Hereinafter, although the present invention will be described in more detail by Examples, the present invention is not limited to these Examples.

[Experimental Example 1]

(1) Preparation of protein expression strain (recombinant cell) to be targeted

A nucleic acid encoding modified spider silk fibroin (PRT799) having an amino acid sequence set forth in ■ SEQ ID NO: 15 was synthesized. In the nucleic acids, an NdeI site was added to the 5'-terminus and an EcoRI site was added to a termination codon downstream.

Each of the nucleic acids was cloned with a cloning vector (pUC118). Thereafter, the same nucleic acids were cleaved by restriction enzyme treatment with NdeI and EcoRI, and then recombined into a protein expression vector pET-22b (+) to obtain an expression vector. Transformed E. coli (recombinant cell) expressing a protein was to be targeted obtained by transforming E. coli BLR (DE3) with each pET-22b (+) expression vector in which each of the nucleic acids was recombined.

(2) Expression of protein to be targeted

The transformed E. coli was cultured in a 2 mL LB medium containing ampicillin for 15 hours. The culture solution was added to a 100 mL seed culture medium containing ampicillin (Table 4) so that $OD_{600}$ was 0.005. The culture solution temperature was maintained at 30°C, and flask culture was performed until $OD_{600}$ reached 5 (for about 15 hours) to obtain a seed culture solution.

[Table 4]

| Seed culture medium (at the start of culturing, per 1 L) | |
| --- | --- |
| Glucose | 5 g |
| $KH_2PO_4$ | 4 g |
| $K_2HPO_4$ | 10 g |
| Yeast Extract | 6 g |

Ampicillin was added so that the final concentration was 100 mg/L to be used as a seed culture medium.

The seed culture solution was added to a jar fermenter to which a 500 mL production medium (Table 5) was added so that $OD_{600}$ was 0.05. The culture was performed while maintaining the culture solution temperature at 37°C and constantly controlling the pH to 6.9. In addition, a dissolved oxygen concentration in the culture solution was set to be maintained at 20% of a dissolved oxygen saturation concentration.

[Table 5]

| Production medium (at the start of culturing, per 1 L) | |
| --- | --- |
| Glucose | 12 g |
| $KH_2PO_4$ | 9 g |
| $MgSO_4 \cdot 7H_2O$ | 2.4 g |
| Yeast Extract | 15 g |
| $FeSO_4 \cdot 7H_2O$ | 40 mg |
| $MnSO_4 \cdot 5H_2O$ | 40 mg |
| $CaCl_2 \cdot 2H_2O$ | 40 mg |
| GD-113 (Antifoaming agent) | 0.1 mL |

Immediately after glucose in the production medium was completely consumed, a feed solution (glucose 455 g/1 L, yeast extract 120 g/1 L) was added at a rate of 1 mL/min. The culture was performed while maintaining the culture solution temperature at 37°C and constantly controlling the pH to 6.9. In addition, the culture was performed for 20 hours while maintaining a dissolved oxygen concentration in the culture solution at 20% of a dissolved oxygen saturation concentration. Thereafter, 1 M isopropyl-β-thiogalactopyranoside (IPTG) was added to the culture solution so that the final concentration was 1 mM to induce expression of the target protein. 20 hours after the addition of IPTG, the culture solution was centrifuged to collect fungus bodies. SDS-PAGE was performed by using fungus bodies prepared from the culture solution before the addition of IPTG and the culture solution after the addition of IPTG, and expression of the protein to be targeted as an insoluble matter was confirmed by appearance of a band with a size of the protein to be targeted depending on the addition of IPTG.

(3) Purification of protein

2 hours after the addition of IPTG, the collected fungus bodies were washed with a 20 mM Tris-HCl buffer (pH 7.4). The washed fungus bodies were suspended in a 20 mM Tris-HCl buffer (pH 7.4) containing approximately 1 mM PMSF, and cells were disrupted in a high-pressure homogenizer (manufactured by GEA Niro Soavi Technologies). The disrupted cells were centrifuged to obtain a precipitate. The obtained precipitate was washed with a 20 mM Tris-HCl buffer (pH 7.4) until the purity was high. The washed precipitate was suspended in an 8 M guanidine buffer (8 M guanidine hydrochloride, 10 mM sodium dihydrogen phosphate, 20 mM NaCl, and 1 mM Tris-HCl, pH 7.0) so that a concentration thereof was 100 mg/mL, and the suspended precipitate was dissolved by performing stirring using a stirrer at 60°C for 30 minutes. After dissolution, the resultant product was dialyzed with water by using a dialysis tube (cellulose tube 36/32 manufactured by Sanko Junyaku Co., Ltd.). A white coagulation protein obtained after dialysis was collected by centrifugation, and water was removed in a freeze dryer to recover freeze-dried powdered protein.

(4) Molding of protein fiber

The dry power of modified spider silk fibroin was dissolved in formic acid to obtain a dope solution (a protein concentration in the dope solution: 25% by mass). The dope solution was discharged to a coagulation liquid (methanol) by a gear pump using a known spinning apparatus. Spinning conditions were as below. As a result, a protein fiber (fibroin fiber) was obtained.

(Spinning conditions)

    Dope solution temperature: 25°C
    Hot roller temperature: 60°C

(5) Hydrolysis treatment

A hydrolysis treatment was performed by the following steps i to iv.

    i. The protein fiber was immersed and stirred in an acidic or basic aqueous solution for various times.
    ii. The protein fiber was immersed and stirred in an excess amount of pure water for 2 minutes.
    iii. The pure water was replaced, and the operation of ii was further performed twice more.
    iv. The protein fiber was air-dried at room temperature.

A pH and reaction time of the acidic or basic aqueous solution used in each of Examples and Comparative Examples are shown in Table 6.

(6) Confirmation of reduction behavior of formic acid ester group by FT-IR

A reduction behavior of a formic acid ester group was confirmed by measuring each fiber dried through the hydrolysis treatment by FT-IR transmission microscopy. As for each sample treated under each of the pH conditions, a peak height ratio P1/P2 was determined and recorded. In this case, $(P1/P2) \leq 0.01$ was used as a determination criterion for completion of removal. The results are shown in Table 6. P1 and P2 are peak heights corresponding to subsequent wave numbers. The smaller the value of the absorbance ratio P1/P2, the smaller the number of the structural ester groups.

    P1: a peak height of 1,725 cm$^{-1}$ (peak based on C=O of ester)
    P2: a peak height of 1,445 cm$^{-1}$ (peak based on amide III of protein)

(7) Retention rate of elongation

Each fiber dried through the hydrolysis treatment was fixed with an adhesive to a piece of a test paper having a distance between gripping tools of 20 mm, and a stress (strength) and elongation were measured under conditions of a temperature of 20°C and a relative humidity of 65% at a tensile speed of 10 cm/min using a tensile tester 3342 manufactured by Instron Co., Ltd. The load cell capacity was 10 N and the gripping tool was a clip type. The elongation of the protein fiber treated with the acidic or basic aqueous solution and the elongation of the protein fiber treated with water having a pH of 7 were measured to calculate a retention rate of the elongation according to the following equation. The results are shown in Table 6.

[Retention rate of elongation] = [elongation of protein fiber treated with acidic or basic aqueous solution]/[elongation of protein fiber treated with water having pH of 7] $\times$ 100

[Table 6]

|  | pH | Immersion time [min] | Absorbance ratio (P1/P2) of formic acid ester | Retention rate [%] of elongation |
|---|---|---|---|---|
| Example 1-1 | 1 | 180 (70 deg) | 0.00 | 99 |
| Example 1-2 | 8 | 2400 (r.t.) | 0.00 | 94 |
| Example 1-3 | 10 | 80 (r.t.) | 0.00 | 101 |
| Example 1-4 | 11 | 20 (r.t.) | 0.00 | 99 |
| Example 1-5 | 12 | 10 (r.t.) | 0.00 | 55 |
| Comparative Example 1-1 | 7 | 90 (r.t.) | 0.09 | 100 |

The absorbance ratio of formic acid ester was reduced in the protein fiber treated with the acidic or basic aqueous solution. From the results, it was shown that the ester group included in the protein fiber was hydrolyzed by the treatment with the acidic or basic aqueous solution, and the ester group was thus removed. In addition, when the pH of the acidic or basic aqueous solution is 11 or lower (lower than 12), the elongation of the treated protein fiber was also maintained.

(8) Evaluation of crimp property

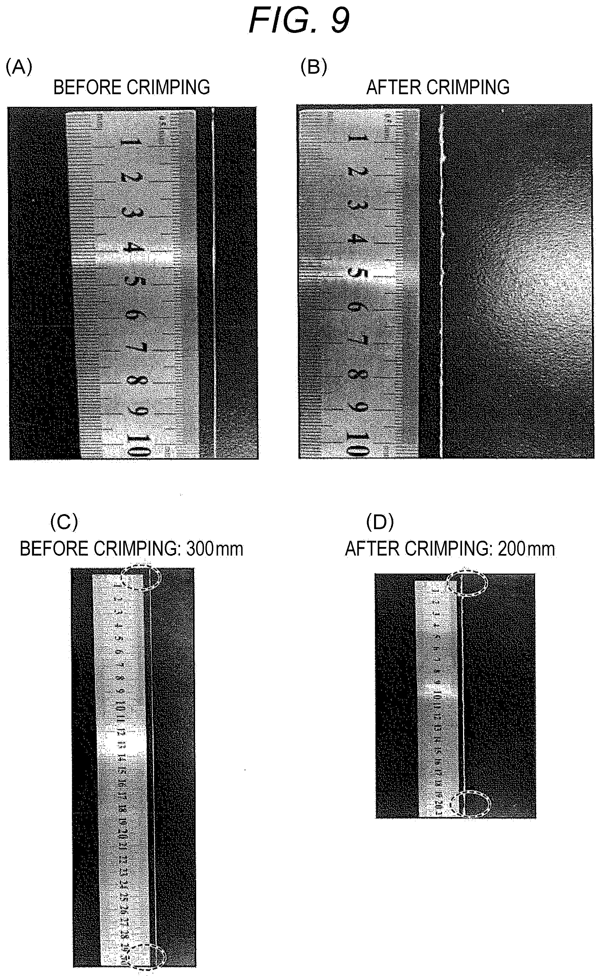

The hydrolysis treatment was performed on the protein fiber under the conditions (pH and immersion time) of Example 1-2, and the crimped states of the protein fiber before the hydrolysis treatment and the protein fiber after the hydrolysis treatment were compared. The crimped states were further compared by measuring each of the protein fibers with a ruler. The results are illustrated in Fig. 9.
Figs. 9(A) and (C) illustrate photographs of the protein fibers before the hydrolysis treatment (before crimping). Figs. 9(B) and (D) illustrate photographs of the protein fibers after the hydrolysis treatment (after crimping). The 300 mm protein fiber before the hydrolysis treatment became 200 mm (Figs. 9(C) and (D)). In addition, coupled with Figs. 9(A) and (B), it could be seen that the protein fiber after the hydrolysis treatment was crimped as compared to the protein fiber before the hydrolysis treatment.

(9) Evaluation of shrink-proof property

The protein fiber (200 mm) after the hydrolysis treatment was not shrunk any further even in a case where the protein fiber was immersed in water.
On the other hand, when the protein (300 nm) before the hydrolysis treatment was immersed in water as Comparative Example 1-1, the protein became 200 mm. Therefore, it could be seen that the protein fiber after the hydrolysis treatment was shrink-proofed as compared with the protein fiber before the hydrolysis treatment.

(10) Hydrolysis by water vapor

The protein fiber was allowed to stand under conditions of humidity of 80% and 60°, and a daily absorbance ratio P1/P2 was measured using FT-IR (manufactured by NICOLET Co., Ltd., FT-IR iS50). The results are shown in Table 7.

[Table 7]

| Treatment time [day] (60°C, 80%) | P1: 1,725 cm⁻¹ Ester C=O stretch | P2: 1,445 cm⁻¹ Artificial protein amide III | Absorbance ratio (P1/P2) |
|---|---|---|---|
| 0 | 0.0017 | 0.0230 | 0.074 |
| 1 | 0.0020 | 0.0320 | 0.063 |
| 2 | 0.0015 | 0.0280 | 0.054 |
| 3 | 0.0017 | 0.0380 | 0.045 |
| 4 | 0.0015 | 0.0280 | 0.054 |
| 5 | 0.0016 | 0.0310 | 0.052 |
| 6 | 0.0012 | 0.0330 | 0.036 |

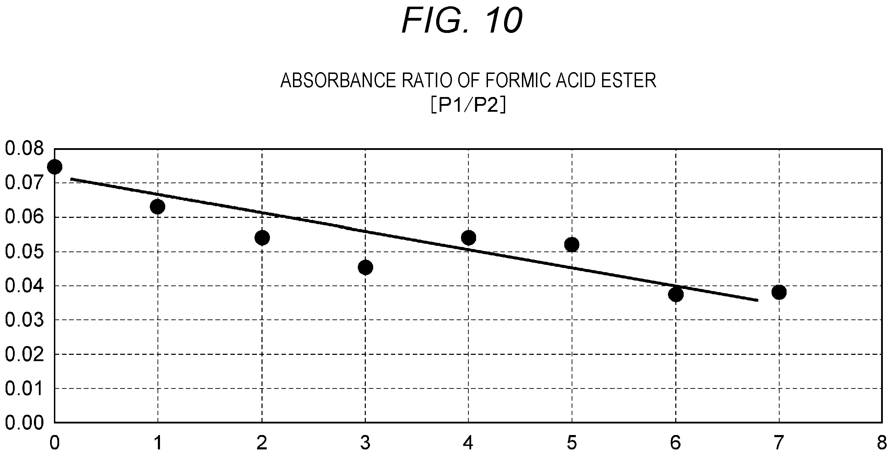

Fig. 10 is a diagram obtained by plotting data of Table 7 with the absorbance ratio P1/P2 on the vertical axis and the treatment time (unit: day) on the horizontal axis.

As shown in Table 7 and illustrated in Fig. 10, when the protein fiber was brought into contact with water, the absorbance ratio P1/P2 was reduced. From the results, it was shown that the ester hydrolysis reaction proceeded by contact with water using the remaining formic acid as a catalyst, and the ester group added to the protein fiber was reduced.

[Experimental Example 2]

(1) Preparation of expression vector

Spider silk fibroin having ■ SEQ ID NO: 40 (hereinafter, also referred to as "PRT966") was designed based on a base sequence and an amino acid sequence of fibroin derived from Nephila clavipes (GenBank Accession No.: P46804.1, GI: 1174415). The amino acid sequence set forth in SEQ ID NO: 40 has a sequence obtained by substituting, with VF, all QQs in a sequence obtained by repeating a region of 20 domain sequences present in an amino acid sequence set forth in SEQ ID NO: 7 two times and substituting the remaining Q with I, for the purpose of improving hydrophobicity, and is obtained by adding an amino acid sequence (tag sequence and hinge sequence) set forth in SEQ ID NO: 11 to the N-terminus.

Subsequently, a nucleic acid encoding the designed protein (spider silk fibroin) was synthesized to obtain transformed E. coli (recombinant cell) expressing a protein to be targeted in the same manner as that of Experimental Example 1.

(2) Expression of protein

The transformed E. coli was cultured in a 2 mL LB medium containing ampicillin for 15 hours. The culture solution was added to a 100 mL seed culture medium containing ampicillin (Table 8) so that $OD_{600}$ was 0.005. The culture solution temperature was maintained at 30°C, and flask culture was performed until $OD_{600}$ reached 5 (for about 15 hours) to obtain a seed culture solution.

[Table 8]

| Seed culture medium | |
|---|---|
| Reagent | Concentration (g/L) |
| Glucose | 5.0 |
| $KH_2PO_4$ | |
| $K_2HPO_4$ | 4.0 9.3 |
| Yeast Extract | 6.0 |
| Ampicillin | 0.1 |

The seed culture solution was added to a jar fermenter to which a 500 ml production medium (Table 5 of Experimental Example 1) was added so that $OD_{600}$ was 0.05. The culture was performed while maintaining the culture solution temperature at 37°C and constantly controlling the pH to 6.9. In addition, a dissolved oxygen concentration in the culture solution was set to be maintained at 20% of a dissolved oxygen saturation concentration.

Immediately after glucose in the production medium was completely consumed, a feed solution (glucose 455 g/1 L, Yeast Extract 120 g/1 L) was added at a rate of 1 mL/min. The culture was performed while maintaining the culture solution temperature at 37°C and constantly controlling the pH to 6.9. In addition, the culture was performed for 20 hours so that a dissolved oxygen concentration in the culture solution was set to be maintained at 20% of a dissolved oxygen saturation concentration. Thereafter, 1 M isopropyl-β-thiogalactopyranoside (IPTG) was added to the culture solution so that the final concentration was 1 mM to induce expression of the modified fibroin. 20 hours after the addition of IPTG, the culture solution was centrifuged to collect fungus bodies. SDS-PAGE was performed by using fungus bodies prepared from the culture solution before the addition of IPTG and the culture solution after the addition of IPTG, and expression of the protein (spider silk fibroin) to be targeted was confirmed by appearance of a band of the recombinant protein to be targeted depending on the addition of IPTG.

(3) Purification of protein

The protein was purified by the same method as in Experimental Example 1 to recover freeze-dried powdered protein (dry powder of spider silk fibroin).

(4) Production of raw material fiber

The dry powder of spider silk fibroin was dissolved in formic acid, filtration was performed with a metal filter having a mesh size of 1 μm, thereby obtaining a dope solution (a concentration of the protein in the dope solution: 30% by mass). The dope solution was discharged to a coagulation liquid by a gear pump using a known spinning apparatus. Spinning conditions were as below. Therefore, a raw material fiber (raw material spider silk fibroin fiber) was obtained.

(Spinning conditions)

Nozzle hole diameter: 0.1 mm
Coagulation liquid: methanol
Temperature of coagulant liquid: 25°C
Temperature of water washing bath: 25°C
Hot roller temperature: 60°C

(5) Ester hydrolysis treatment of raw material fiber (production of protein fiber)

200 g of the raw material fiber obtained in (4) was formed into a hank state and a hydrolysis treatment was performed by the following steps [i] to [x] using a known hank treatment machine. The treatment time, treatment temperature, and treatment medium in the step [iii] are shown in Table 9. The step [v] is a neutralization treatment step performed when a basic medium is used as the treatment medium. The steps [vii] and [viii] may be appropriately performed, if necessary. In addition, an agent for removing an oil agent in the treatment medium shown in Table 9 is added for removing the oil agent added to a surface of the fiber, and the agent may be appropriately used, if necessary.

[i] A hank was set to the hank treatment machine.
[ii] The hank was washed with tap water.
[iii] The hank was impregnated with the treatment medium shown in Table 9 and stirring was performed. The treatment time is as shown in Table 9.
[iv] The hank was washed by replacing the tap water.
[v] The hank was subjected to a neutralization treatment in a 1% aqueous acetic acid solution.
[vi] The hank was washed by replacing the tap water.
[vii] The hank was impregnated with an aqueous softener and stirring was performed.
[viii] The hank was washed by replacing the tap water.
[ix] The hank was dehydrated.
[x] The hank was air-dried at 50 to 60°C for 2 hours.

[Table 9]

| | Treatment conditions of hydrolysis treatment | | | Formic acid ester ($V_{C=O}$ 1,730 cm$^{-1}$) | Elongation [%] | Standard deviation [σ] | Relative value [%] of elongation |
|---|---|---|---|---|---|---|---|
| | Temperature [°C] | Treatment time [min] | Treatment medium | | | | |
| Example 2-1 | 40 | 60 | Aqueous solution of Na$_2$CO$_3$ with pH 11 | Undetectable | 82.3 | 3.25 | 127 |
| Example 2-2 | 90 | 15 | Aqueous solution of Na$_2$CO$_3$ with pH 11 Agent for removing oil agent | Undetectable | 88.2 | 1 | 136 |
| Example 2-3 | 98 | 15 | Aqueous solution of Na$_2$CO$_3$ with pH 11 Agent for removing oil agent | Undetectable | 89.1 | 3.44 | 138 |
| Comparative example 2-1 | - | 0 | None (untreated) | Detect | 64.8 | 11.4 | 100 |
| Comparative example 2-2 | 90 | 15 | Aqueous solution of agent for removing oil agent | Detect | 81.3 | 3.54 | 125 |
| Comparative example 2-3 | 98 | 15 | Aqueous solution of agent for removing oil agent | Detect | 81.5 | 2.13 | 126 |

(6) Evaluation of removal of ester group by FT-IR

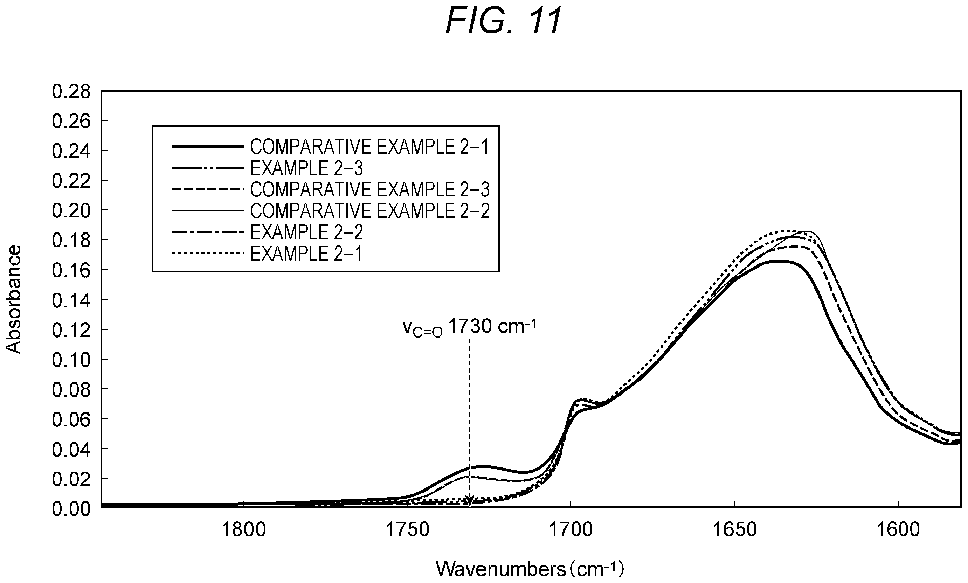

Each fiber obtained in (5) was measured by an ATR method (total reflection method) using a Fourier-transform infrared spectrophotometer to evaluate the removal of the formic acid ester group. In each of fibers (Examples 2-1 to 2-3 and Comparative Examples 2-1 to 2-3), a height of a peak (peak assigned to C=O of the ester group) with a wave number of 1,730 cm$^{-1}$ was confirmed from an IR spectrum to evaluate whether or not the peak was detected (Fig. 11). A case where the peak was not detected was determined that the formic acid ester group was removed.

(7) Evaluation of elongation

Each fiber obtained in (5) was fixed with an adhesive to a piece of a test paper having a distance between gripping tools of 20 mm, and elongation was measured under conditions of a temperature of 20°C and a relative humidity of 65% at a tensile speed of 10 cm/min using a tensile tester 3342 manufactured by Instron Co., Ltd (Table 9). The load cell capacity was 10 N and the gripping tool was a clip type.

As shown in Table 9, it was confirmed that the hydrolysis reaction of the formic acid ester group proceeded and the formic acid ester group in the fiber was removed only in a case where the raw material fiber was treated with the medium (basic aqueous solution) containing basic water (Examples 2-1 to 2-3). Furthermore, it was shown that the formic acid ester group was hydrolyzed and removed in a shorter time by setting the treatment temperature to a high temperature (90°C to 98°C, Examples 2-2 and 2-3). Productivity in the hydrolysis step was significantly improved by performing the hydrolysis treatment to form the fiber into a hank state. In addition, it was confirmed that the elongation was not reduced by the hydrolysis treatment.

(8) Evaluation of shrinkability to water

Shrinkability (dimensional stability) of the protein fiber after the ester hydrolysis treatment obtained in (5) to water was evaluated by the following procedure. The shrinkability to water was calculated and evaluated according to the following Equation (1) as a shrinkage rate. The calculation values are shown in Table 10. The shrinkability was evaluated using a fiber which was not subjected to the ester hydrolysis treatment (Comparative Example 2-1) for comparison in the same manner as above.

A plurality of fibers obtained in (5) were bundled to a length of about 30 cm to obtain a fiber bundle having a fineness of 150 denier. The length of the fiber bundle was used as "length of protein fiber before in wet state". The fiber bundle was immersed (wetted) in water at 90°C for 15 minutes, and the immersed fiber bundle was dried at room temperature for 2 hours to measure a length thereof. The length was used as "length of protein fiber when dried from wet state" to calculate a shrinkage rate according to the following Equation (1). A measured value was an average value of the number of samples (n = 3).

Shrinkage rate = {1 - (length of protein fiber when dried from wet state/length of protein fiber before in wet state)} $\times$ 100 [%].

[Table 10]

| | Treatment conditions of hydrolysis treatment | | | Shrinkage rate [%] |
|---|---|---|---|---|
| | Temperature [°C] | Treatment time [min] | Treatment medium | |
| Example 2-2 | 90 | 15 | Aqueous solution of $Na_2CO_3$ with pH 11 Agent for removing oil agent | 3 |
| Comparative example 2-1 | - | - | None (untreated) | 40 |

As shown in Table 10, it was confirmed that in the case of the protein fiber which was subjected to the ester hydrolysis treatment (Example 2-2), the shrinkage rate was reduced as compared to that of the protein fiber which was not subjected to the ester hydrolysis treatment (Comparative Example 2-1), and the shrinkability to water was reduced. It was shown that the shrink-proof effect was obtained by simultaneously performing the shrink-proof (shrink) treatment through the ester hydrolysis treatment.

Reference Signs List

1      Extrusion device
2      Undrawn yarn production apparatus
3      Wet heat drawing device
4      Drying device
6      Dope solution
10     Spinning apparatus
20     Coagulation bath
21     Drawing bath
25     Spinning apparatus
36     Raw material fiber
38     Protein fiber
40     Production apparatus
42     Feed roller
44     Winder

46      Water bath
48      Dryer
54      Heater
56      Tension roller
58      Hot roller
60      Processing device
62      Drying device
64      Dry heat plate
140     Relaxation shrinking means (heating means)
141     Feed means
142     Winding means
146     Speed control means
147     Temperature control means

**Claims**

1. A production method of a protein composition, the method comprising a step of hydrolyzing an ester group by bringing a raw material composition containing an esterified protein into contact with an acidic or basic medium,

   wherein the medium is a medium containing water, and the medium containing water is 40°C to 180°C,
   wherein the medium containing water is an aqueous solution or water vapor, and
   wherein the protein is a structural protein.

2. The production method of a protein composition according to claim 1, wherein the medium containing water is an aqueous solution, and a temperature of the aqueous solution is 40°C or higher and a boiling point or lower.

3. The production method of a protein composition according to claim 2, wherein the aqueous solution is a basic aqueous solution having a pH of 12 or lower.

4. The production method of a protein composition according to any one of claims 1 to 3, wherein the structural protein is fibroin.

5. The production method of a protein composition according to claim 4, wherein the fibroin is spider silk fibroin.

6. The production method of a protein composition according to any one of claims 1 to 5, wherein the esterified protein contains formic acid ester.

7. The production method of a protein composition according to any one of claims 1 to 6, wherein the raw material composition is at least one selected from the group consisting of a fiber, a film, a molded article, a gel, a porous body, and a particle.

8. The production method of a protein composition according to any one of claims 1 to 7, wherein the raw material composition is a fiber, the medium containing water is an aqueous solution, and the production method further comprises:

   (a) a crimping step of crimping the fiber by bringing the fiber into contact with the aqueous solution; or
   (b) a shrink-proof step of shrink-proofing the fiber by bringing the fiber into contact with the aqueous solution.

9. The production method of a protein composition according to claim 8, wherein the raw material composition is a fiber, and the fiber is:

   (a) a hank state; or
   (b) a cloth state.

**Patentansprüche**

1. Herstellungsverfahren einer Proteinzusammensetzung, wobei das Verfahren einen Schritt des Hydrolysierens einer Estergruppe durch Inkontaktbringen einer Rohmaterialzusammensetzung, die ein verestertes Protein enthält, mit einem sauren oder basischen Medium umfasst,

wobei das Medium ein wasserhaltiges Medium ist und das wasserhaltige Medium 40 °C bis 180 °C ist,
wobei das wasserhaltige Medium eine wässrige Lösung oder Wasserdampf ist, und
wobei das Protein ein Strukturprotein ist.

2. Herstellungsverfahren einer Proteinzusammensetzung nach Anspruch 1, wobei das wasserhaltige Medium eine wässrige Lösung ist und eine Temperatur der wässrigen Lösung 40 °C oder höher und ein Siedepunkt oder niedriger ist.

3. Herstellungsverfahren einer Proteinzusammensetzung nach Anspruch 2, wobei die wässrige Lösung eine basische wässrige Lösung mit einem pH von 12 oder niedriger ist.

4. Herstellungsverfahren einer Proteinzusammensetzung nach einem der Ansprüche 1 bis 3, wobei das Strukturprotein Fibroin ist.

5. Herstellungsverfahren einer Proteinzusammensetzung nach Anspruch 4, wobei das Fibroin Spinnenseidenfibroin ist.

6. Herstellungsverfahren einer Proteinzusammensetzung nach einem der Ansprüche 1 bis 5, wobei das veresterte Protein Ameisensäureester enthält.

7. Herstellungsverfahren einer Proteinzusammensetzung nach einem der Ansprüche 1 bis 6, wobei die Rohmaterialzusammensetzung mindestens eines, ausgewählt aus der Gruppe bestehend aus einer Faser, einem Film, einem Formteil, einem Gel, einem porösen Körper und einem Partikel, ist.

8. Herstellungsverfahren einer Proteinzusammensetzung nach einem der Ansprüche 1 bis 7, wobei die Rohmaterialzusammensetzung eine Faser ist, das wasserhaltige Medium eine wässrige Lösung ist und das Herstellungsverfahren ferner umfasst:

(a) einen Kräuselungsschritt des Kräuselns der Faser, indem die Faser in Kontakt mit der wässrigen Lösung gebracht wird; oder
(b) einen schrumpffesten Schritt des Schrumpffestmachens der Faser, indem die Faser in Kontakt mit der wässrigen Lösung gebracht wird.

9. Herstellungsverfahren einer Proteinzusammensetzung nach Anspruch 8, wobei die Rohmaterialzusammensetzung eine Faser ist und die Faser Folgendes ist:

(a) ein Strangzustand; oder
(b) ein Tuchzustand.

**Revendications**

1. Procédé de production d'une composition protéique, le procédé comprenant une étape d'hydrolyse d'un groupe ester par mise en contact d'une composition de matière première contenant une protéine estérifiée avec un milieu acide ou basique,

dans lequel le milieu est un milieu contenant de l'eau, et le milieu contenant de l'eau est de 40 °C à 180 °C,
dans lequel le milieu contenant de l'eau est une solution aqueuse ou de la vapeur d'eau, et
dans lequel la protéine est une protéine structurale.

2. Procédé de production d'une composition protéique selon la revendication 1, dans lequel le milieu contenant de l'eau est une solution aqueuse, et une température de la solution aqueuse est de 40 °C ou plus et inférieure ou égale au point d'ébullition.

3. Procédé de production d'une composition protéique selon la revendication 2, dans lequel la solution aqueuse est une solution aqueuse basique présentant un pH de 12 ou moins.

4. Procédé de production d'une composition protéique selon l'une quelconque des revendications 1 à 3, dans lequel la protéine structurale est la fibroïne.

5. Procédé de production d'une composition protéique selon la revendication 4, dans lequel la fibroïne est la fibroïne de soie d'araignée.

6. Procédé de production d'une composition protéique selon l'une quelconque des revendications 1 à 5, dans lequel la protéine estérifiée contient un ester d'acide formique.

7. Procédé de production d'une composition protéique selon l'une quelconque des revendications 1 à 6, dans lequel la composition de matière première est au moins l'un choisi dans le groupe constitué d'une fibre, d'un film, d'un article moulé, d'un gel, d'un corps poreux et d'une particule.

8. Procédé de production d'une composition protéique selon l'une quelconque des revendications 1 à 7, dans lequel la composition de matière première est une fibre, le milieu contenant de l'eau est une solution aqueuse, et le procédé de production comprend en outre :

   (a) une étape de frisage consistant à friser la fibre en mettant la fibre en contact avec la solution aqueuse ; ou
   (b) une étape de traitement anti-rétrécissement consistant à conférer un caractère anti-rétrécissant à la fibre en mettant la fibre en contact avec la solution aqueuse.

9. Procédé de production d'une composition protéique selon la revendication 8, dans lequel la composition de matière première est une fibre, et la fibre est :

   (a) à l'état écheveau ; ou
   (b) à l'état tissu.

FIG. 1

EP 3 858 851 B1

# FIG. 2

FIG. 3

REGION A

FIG. 4

## FIG. 5

EP 3 858 851 B1

# FIG. 6

(a)

(b)

FIG. 7

*FIG. 8*

# FIG. 9

(A)
BEFORE CRIMPING

(B)
AFTER CRIMPING

(C)
BEFORE CRIMPING: 300 mm

(D)
AFTER CRIMPING: 200 mm

# FIG. 10

ABSORBANCE RATIO OF FORMIC ACID ESTER
[P1/P2]

EP 3 858 851 B1

FIG. 11

EP 3 858 851 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02052099 A
- JP 2007177370 A
- WO 2012165476 A
- EP 0488687 A2
- JP 2004503204 A
- JP 2002238569 A
- JP 6077570 B
- JP 6077569 B
- JP 5584932 B
- JP 5678283 B
- JP 2017539869 A
- JP 2016076500 W
- JP 05782580 B
- JP 5796147 B

**Non-patent literature cited in the description**

- *Nucleic Acid Res.*, 1982, vol. 10, 6487
- *Methods in Enzymology*, 1983, vol. 100, 448
- GenBank, AAC47010
- GenBank, AAC47011
- GenBank, AAC04504
- GenBank, ABR68856
- GenBank, AAL32472
- GenBank, CAJ00428
- GenBank, CAM32249.1
- GenBank, AAC14589.1
- GenBank, AAC14591.1
- GenBank, ABR37278.1
- **KYTE J** ; **DOOLITTLE R**. A simple method for displaying the hydropathic character of a protein. *J. Mol. Biol.*, 1982, vol. 157, 105-132
- *Proc. Natl. Acad. Sci. USA*, 1972, vol. 69, 2110